# EUROPEAN PATENT SPECIFICATION

(11) **EP 3 455 198 B1**
(45) Date of publication and mention of the grant of the patent: **21.10.2020**
(21) Application number: 17721751.0
(22) Date of filing: 11.05.2017
(51) Int. Cl.: C07C 29/141, C07C 29/145, C07C 33/02, C07C 33/14, C07C 33/20, C07C 33/22, C07C 33/32, C07D 307/42, C07D 307/44, C07D 307/83, B01J 31/02, B01J 31/22, B01J 31/24

(54) **SELECTIVE REDUCTION OF ALDEHYDES AND KETONES**
SELEKTIVE REDUKTION VON ALDEHYDEN UND KETONEN
RÉDUCTION SÉLECTIVE D'ALDÉHYDES ET DE CÉTONES

(30) Priority: 13.05.2016 WO PCT/EP2016/169508; 23.01.2017 WO PCT/EP2017/152590
(43) Date of publication of application: 20.03.2019
(73) Proprietor: DSM IP Assets B.V., 6411 TE Heerlen (NL)
(72) Inventor: BELLER, Matthias, 18059 Rostock (DE); BONRATH, Werner, 4303 Kaiseraugst (CH); DE VRIES, Johannes Gerardus, 18059 Rostock (DE); FAN, Yuting, 18059 Rostock (DE); HÜBNER, Sandra, 18059 Rostock (DE); LEFORT, Laurent, 4303 Kaiseraugst (CH); MEDLOCK, Jonathan Alan, 4303 Kaiseraugst (CH); PUYLAERT, Pim, 18059 Rostock (DE); VAN HECK, Richard, 18059 Rostock (DE)
(74) Representative: Kurt, Manfred
(86) International application number: PCT/EP2017/061298
(87) International publication number: WO 2017/194662

(56) References cited:
- SINGH R ET AL: "Iron(III) complexes using NNS reduced Schiff bases and NNOS coordinating tetradentate ligands: Synthesis, structure and catecholase activity", INORGANICA CHIMICA ACTA, ELSEVIER BV, NL, vol. 363, no. 12, 15 October 2010 (2010-10-15), pages 3131-3138, XP027355274, ISSN: 0020-1693 [retrieved on 2010-05-24]
- SUNGHEE KIM ET AL: "Tuning of the Copper-Thioether Bond in Tetradentate N3S(thioether) Ligands; O-O Bond Reductive Cleavage via a [Cu II 2 ([mu]-1,2-peroxo)]2+/[Cu III 2 ([mu]-oxo)2]2+ Equilibrium", JOURNAL OF THE AMERICAN CHEMICAL SOCIETY, vol. 136, no. 22, 22 May 2014 (2014-05-22) , pages 8063-8071, XP055390366, US ISSN: 0002-7863, DOI: 10.1021/ja502974c
- DONG-HEON LEE ET AL: "Copper(I) Complex O2 -Reactivity with a N3S Thioether Ligand: a Copper-Dioxygen Adduct Including Sulfur Ligation, Ligand Oxygenation, and Comparisons with All Nitrogen Ligand Analogues", INORGANIC CHEMISTRY, vol. 46, no. 15, 20 June 2007 (2007-06-20) , pages 6056-6068, XP055390374, EASTON, US ISSN: 0020-1669, DOI: 10.1021/ic700541k
- MAGDALENA M. MAKOWSKA-GRZYSKA ET AL: "Modeling Substrate- and Inhibitor-Bound Forms of Liver Alcohol Dehydrogenase: Chemistry of Mononuclear Nitrogen/Sulfur-Ligated Zinc Alcohol, Formamide, and Sulfoxide Complexes", INORGANIC CHEMISTRY, vol. 41, no. 19, 20 August 2002 (2002-08-20), pages 4872-4887, XP055390381, EASTON, US ISSN: 0020-1669, DOI: 10.1021/ic0255609
- NEVA LAZAROVA ET AL: "Thiol- and Thioether-Based Bifunctional Chelates for the {M(CO)3}+ Core (M = Tc, Re)", INORGANIC CHEMISTRY, vol. 44, no. 19, 17 August 2005 (2005-08-17), pages 6763-6770, XP055390402, EASTON, US ISSN: 0020-1669, DOI: 10.1021/ic050735a
- LEI TANG ET AL: "A new chiral sulfinylNHpyridine ligand for Ir-catalyzed asymmetric transfer hydrogenation reaction", TETRAHEDRON LETTERS, ELSEVIER, AMSTERDAM, NL, vol. 53, no. 30, 24 April 2012 (2012-04-24), pages 3839-3842, XP028496012, ISSN: 0040-4039, DOI: 10.1016/J.TETLET.2012.04.110 [retrieved on 2012-04-28]
- PIM PUYLAERT ET AL: "Selective Hydrogenation of [alpha],[beta]-Unsaturated Aldehydes and Ketones by Air-Stable Ruthenium NNS Complexes", CHEMISTRY - A EUROPEAN JOURNAL, vol. 23, no. 35, 31 May 2017 (2017-05-31), pages 8473-8481, XP055538816, DE ISSN: 0947-6539, DOI: 10.1002/chem.201700806

## Description

The present invention relates to a selective reduction of specific aldehydes and ketones to their corresponding alcohols.

Reduction of an aldehyde or ketone into the corresponding alcohol is a fundamental and very important reaction in organic chemistry, and it is used in a large number of chemical processes. The obtained alcohols are used as such or are important intermediates in further chemical processes.

A convenient manner to achieve such reduction is to use a hydrogenation process. The hydrogenation process can be carried out with H₂ gas or as a transfer hydrogenation. Therefore, in the context of the present invention the term "hydrogenation" (if not otherwise stated) covers the hydrogenations with H₂ gas as well as the transfer hydrogenations.

Tang et al., Tet. Lett. 53 (2012), p. 3839, shows a chiral sulfinyl-NH-pyridine ligand for use in the iridium-catalyzed asymmetric transfer hydrogenation of acetophenone to 1-phenylethanol.

The specific aldehydes and ketones which are reduced in the context of the present invention are α,β-unsaturated aldehydes and ketones. These aldehydes and ketones have the following general formula (I) 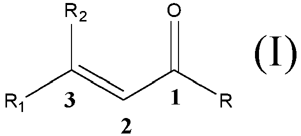
wherein R is H (aldehydes) or an alkyl group (ketones), and
R₁ and R₂ can be a suitable organic moiety (which are defined below).

The problem of the hydrogenation of compounds of formula (I) is that (at least) two sites could be hydrogenated. Either the =O group or the C-C double bond (or both).

Therefore it is possible that a variety of hydrogenated products can be obtained (in any kind of mixture). Mainly the following compounds are obtained:

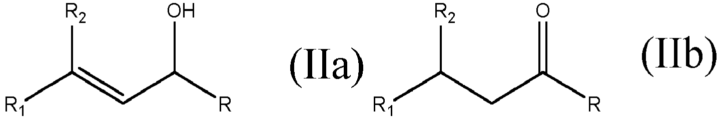

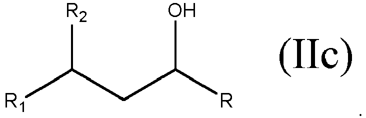

The problem is that when a mixture of such compounds is obtained, a separation step needs to be carried out. Additionally, the yield of the desired alcohol is usually low.

Now, the goal of the present invention was to find a way that a selective reduction (hydrogenation) of specific aldehydes and ketones can be achieved, wherein mainly compounds of the general formula (IIa) are obtained in excellent yield and selectivity.

The specific aldehydes and ketones, which are of interest in the context of the present patent application are those of formula (I) 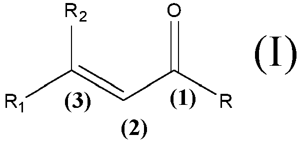 wherein
- R: is H or a C₁-C₄ alkyl group, or R forms together with carbon atom (2) a 4 to 8 membered aliphatic ring system, which can be substituted; and
- R₁: is H; an aromatic ring system which is unsubstituted or an aromatic ring system which is substituted; a heteroaromatic ring system which is unsubstituted or a heteroaromatic ring system which is substituted; an aliphatic ring system which is unsubstituted or an aliphatic ring system which is substituted; a heteroaliphatic ring system which is unsubstituted or a heteroaliphatic ring system which is substituted; -CH₃; -CH₂CH₃; or a C₃ - C₁₀ alkyl group, which can be linear or branched and which can be partially unsaturated (comprising C-C double bond(s));
or R₁ forms together with the C-C double bond of formula (I) a 5 to 8 membered aliphatic ring system, which can be substituted or a 5 to 8 membered aromatic ring system, which can be substituted;
or R₁ forms together with the C-C double bond of formula (I) a 5 to 8 membered heteroaliphatic ring system, which can be substituted or a 5 to 8 membered aromatic ring system, which can be substituted
or R₁ forms together with the C-C double bond of formula (I) a 5 to 8 membered aromatic ring system, which can be substituted or a 5 to 8 membered aromatic ring system, which can be substituted
or R₁ forms together with the C-C double bond of formula (I) a 5 to 8 membered heteroaromatic ring system, which can be substituted or a 5 to 8 membered aromatic ring system, which can be substituted;
or R and R₁ form together a 3 to 8 membered aliphatic ring system, which can be substituted; and
- R₂: is H; -CH₃; -CH₂CH₃; or a C₃ - C₁₀ alkyl group, which can be linear or branched and which can be partially unsaturated (comprising C-C double bond(s)).

The corresponding alcohols, which are the selectively hydrogenated products are those of formula (IIa) 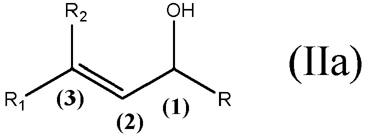 wherein the substituents have the same definition as in formula (I).

Surprisingly it was found that by the use of new specific catalysts it is possible to reduce the compounds of formula (I) selectively in excellent yield and selectivity under mild reaction conditions.

The catalysts, which are used in the selective reduction (hydrogenation) according to the present invention are transition metal catalysts of formula (III)

[M(L)(X)ₐ(L')_{b}] **(III)**,

wherein
M is a transition metal (preferably a transition metal chosen from the group consisting of Os, Co, Ru and Fe, more preferably from the group consisting of Ru and Fe) and
X is an anion (preferably a halogen anion, a carboxylate (such as acetate or benzoate), borohydride (such as BH₄⁻), hydride, BF₄⁻, or PF₆⁻, more preferably a halogen anion, most preferably Cl⁻), and
L' is a monodentate ligand (preferably a monodentate phosphine ligand, more preferably triphenylphosphine (=PPh₃)), and
L is a tridentate ligand (which means that the ligand can be bound to the M at up to three sites) of formula (IV) 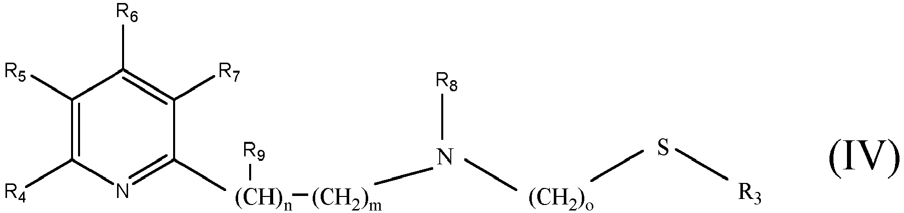 wherein
   R₃ is a linear C₁ - C₄ alkyl group, which can be substituted; a branched C₃ - C₄ alkyl group, which can be substituted; or a phenyl group, which can be substituted, and
   R₄ is H; a linear C₁ - C₄ alkyl group, which can be substituted; a branched C₃ - C₄ alkyl group, which can be substituted; or OC₁-C₂alkyl, and
   R₅ is H; a linear C₁ - C₄ alkyl group, which can be substituted; a branched C₃ - C₄ alkyl group, which can be substituted; or OC₁-C₂alkyl, and
   or R₄ and R₅ form a C₄-C₈ ring system, which can be aliphatic or aromatic, and
   R₆ is H; a linear C₁ - C₄ alkyl group, which can be substituted; a branched C₃ - C₄ alkyl group, which can be substituted; or OC₁-C₂alkyl, and
   R₇ is H; a linear C₁ - C₄ alkyl group, which can be substituted; a branched C₃ - C₄ alkyl group, which can be substituted; or OC₁-C₂alkyl, and
   R₈ is H or a a linear C₁ - C₄ alkyl group, which can be substituted; a branched C₃ - C₄ alkyl group, which can be substituted, and
   R₉ is -CH₃ or -CH₂CH₃, and
   m is 0, 1 or 2, and
   n is 0, 1 or 2,
   with the proviso that the sum of m+n is 1 or 2,
   o is 2 or 3,
   a is 0, 1, 2, or 3,
   b is 0, 1, 2, or 3,
   with the proviso that the sum of a+b is 2, 3 or 4.

From the state of the art, it is known that transition metal complexes can exist as monomers as well as dimers or even as oligomers. The present formula (III) defines the empirical formula of the catalyst.

Therefore the present invention relates to a process (P) of production of a compound of formula (IIa) 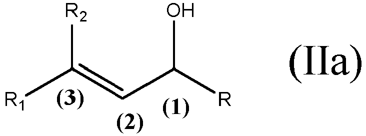 wherein
R is H or a C₁-C₄ alkyl group, or
   R forms together with carbon atom (2) a 4 to 8 membered aliphatic ring system, which can be substituted; and
R₁ is H; an aromatic ring system which is unsubstituted or an aromatic ring system which is substituted; a heteroaromatic ring system which is unsubstituted or a heteroaromatic ring system which is substituted; an aliphatic ring system which is unsubstituted or an aliphatic ring system which is substituted; a heteroaliphatic ring system which is unsubstituted or a heteroaliphatic ring system which is substituted; -CH₃; -CH₂CH₃; or a C₃ - C₁₀ alkyl group, which can be linear or branched and which can be partially unsaturated (comprising C-C double bond(s)) or R₁ forms together with the C-C double bond of formula (IIa) a 5 to 8 membered aliphatic ring system, which can be substituted or a 5 to 8 membered aromatic ring system, which can be substituted;
or R₁ forms together with the C-C double bond of formula (IIa) a 5 to 8 membered heteroaliphatic ring system, which can be substituted or a 5 to 8 membered aromatic ring system, which can be substituted
or R₁ forms together with the C-C double bond of formula (IIa) a 5 to 8 membered aromatic ring system, which can be substituted or a 5 to 8 membered aromatic ring system, which can be substituted
or R₁ forms together with the C-C double bond of formula (IIa) a 5 to 8 membered heteroaromatic ring system, which can be substituted or a 5 to 8 membered aromatic ring system, which can be substituted;
or R and R₁ form together a 3 to 8 membered aliphatic ring system, which can be substituted; and
R₂ is H; -CH₃; -CH₂CH₃; or a C₃ - C₁₀ alkyl group, which can be linear or branched and which can be partially unsaturated (comprising C-C double bond(s))
by a selective reduction of a compound of formula (I) 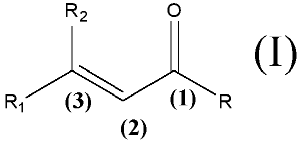 wherein the R, R₁ and R₂ have the same meaning as defined in the compound of formula (IIa),
characterised in, that the selective reduction is carried out in the presence of at least one transition metal catalyst of formula (III)

[M(L)(X)ₐ(L')_{b}] **(III)**,

wherein
M is a transition metal and
X is an anion, and
L' is a monodentate ligand, and
L is a tridentate ligand of formula (IV) 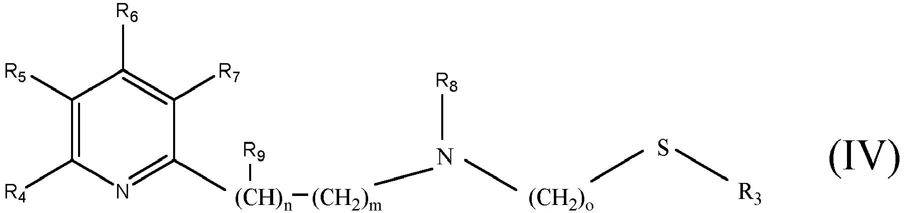 wherein
   R₃ is a linear C₁ - C₄ alkyl group, which can be substituted; a branched C₃ - C₄ alkyl group, which can be substituted; or a phenyl group, which can be substituted, and
   R₄ is H; a linear C₁ - C₄ alkyl group, which can be substituted; a branched C₃ - C₄ alkyl group, which can be substituted; or OC₁-C₂alkyl, and
   R₅ is H; a linear C₁ - C₄ alkyl group, which can be substituted; a branched C₃ - C₄ alkyl group, which can be substituted; or OC₁-C₂alkyl, and
   or R₄ and R₅ form a C₄-C₈ ring system, which can be aliphatic or aromatic, and
   R₆ is H; a linear C₁ - C₄ alkyl group, which can be substituted; a branched C₃ - C₄ alkyl group, which can be substituted;or OC₁-C₂alkyl, and
   R₇ is H; a linear C₁ - C₄ alkyl group, which can be substituted; a branched C₃ - C₄ alkyl group, which can be substituted;or OC₁-C₂alkyl, and
   R₈ is H or a a linear C₁ - C₄ alkyl group, which can be substituted; a branched C₃ - C₄ alkyl group, which can be substituted, and
   R₉ is -CH₃ or -CH₂CH₃, and
   m is 0, 1 or 2, and
   n is 0, 1 or 2,
   with the proviso that the sum of m+n is 1 or 2,
   o is 2 or 3,
   a is 0, 1, 2, or 3,
   b is 0, 1, 2, or 3,
   with the proviso that the sum of a+b is 2, 3 or 4,
   and wherein the process is carried out in the presence of at least one base of formula (VIII)

      M¹(OC₁-C₅alkyl) (VIII),

      wherein M¹ is an alkali metal.

The process according to the present invention is carried out in the presence of at least one base.

Especially preferred bases are selected from the group consisting of KOtBu, NaOtBu and LiOtBu.

Therefore the present invention relates to a process (P1"), which is process (P), wherein the process is carried out in the presence of at least one base of formula (VIII'),

M¹(OC₃-C₅alkyl) (VIII')

wherein
M¹ is Li, Na or K.

Therefore the present invention relates to a process (P1'"), which is process (P), wherein the process is carried out in the presence of at least one base selected from the group consisting of KOtBu, NaOtBu and LiOtBu.
The amount of the base can vary. Usually and preferably the base (or mixture of bases) is used in an amount of 0.1 - 5 mol-% (based on the number of moles of the compound of formula (I)).

Therefore the present invention relates to a process (P1""), which is process (P), (P1") or (P1'"), wherein 0.1 - 5 mol-% (based on the number of moles of the compound of formula (I)) of at least one base is used.

The catalyst of the present invention which is used to selectively reduce the compound of formula (I) is a compound of formula (III) as defined above.

In a preferred embodiment the following catalysts are used:

[M(L)(X)ₐ(L')_{b}] **(III)**,

wherein
M is a transition metal chosen from the group consisting of Os, Co, Ru and Fe, and
X is a halogen anion, a carboxylate (such as acetate or benzoate), borohydride (such as BH₄⁻), hydride, BF₄⁻ or PF₆⁻, and
L' is a monodentate phosphine ligand, and
L is a tridentate ligand of formula (IV) 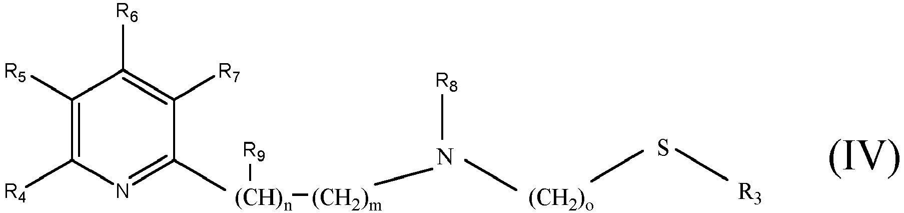 wherein
   R₃ is -CH₃ or -CH₂CH₃, and
   R₄ is H; -CH₃; -CH₂CH₃; -OCH₃ or -OCH₂CH₃, and
   R₅ is H; -CH₃; -CH₂CH₃; -OCH₃ or -OCH₂CH₃, and
   or R₄ and R₅ form a C₄-C₈ ring system, which can be aliphatic or aromatic, and
   R₆ is H; -CH₃; -CH₂CH₃; -OCH₃ or -OCH₂CH₃, and
   R₇ is H; -CH₃; -CH₂CH₃; -OCH₃ or -OCH₂CH₃, and
   R₈ is H; -CH₃ or -CH₂CH₃, and
   R₉ is -CH₃ or -CH₂CH₃, and
   m is 0, 1 or 2, and
   n is 0, 1 or 2,
   with the proviso that the sum of m+n is 1 or 2,
   o is 2 or 3,
   a is 0, 1, 2, or 3,
   b is 0, 1, 2, or 3,
   with the proviso that the sum of a+b is 2 or 3.

In a more preferred embodiment the following catalysts are used:

[M(L)(X)ₐ(L')_{b}] **(III)**,

wherein
M is a transition metal chosen from the group consisting of Ru and Fe, and
X is a halogen anion (preferably Cl⁻), and
L' is triphenylphosphine, and
L is a tridentate ligand of formula (IV) 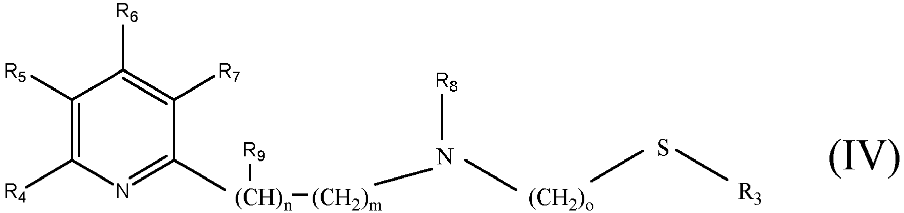 wherein
   R₃ is -CH₃ or -CH₂CH₃, and
   R₄ is H; -CH₃; -CH₂CH₃; -OCH₃ or -OCH₂CH₃, and
   R₅ is H or -CH₃, and
   or R₄ and R₅ form a C₄-C₈ ring system, which can be aliphatic or aromatic, and
   R₆ is H or -CH₃, and
   R₇ is H or -CH₃, and
   R₈ is H or -CH₃, and
   R₉ is -CH₃, and
   m is 0 or 1 and
   n is 0 or 1,
   with the proviso that the sum of m+n is 1,
   o is 2,
   a is 1 or 2,
   b is 1 or 2,
   with the proviso that the sum of a+b is 3.

In an especially preferred embodiment the following catalysts of formula (III')

M(L)(X)₂(L') **(III')**,

wherein
M is Ru or Fe, and
X is Cl⁻, and
L' is PPh₃, and
L is a tridentate ligand chosen from the group consisting of the ligands of formulae (IVa) - (IVI) 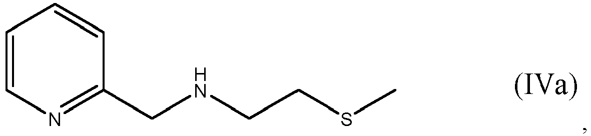 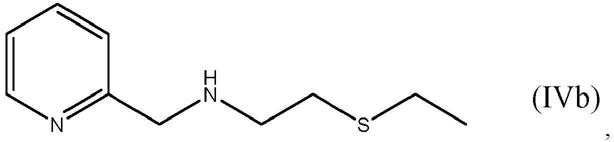 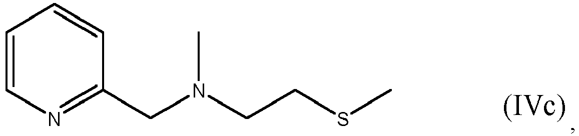 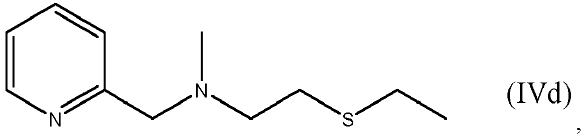 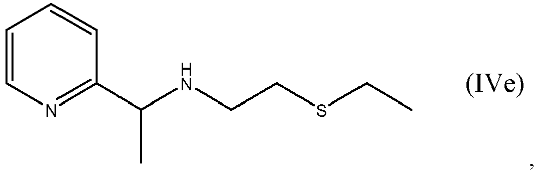 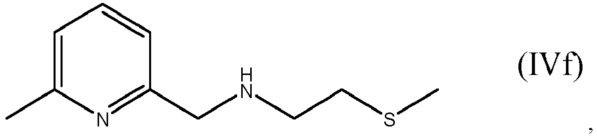 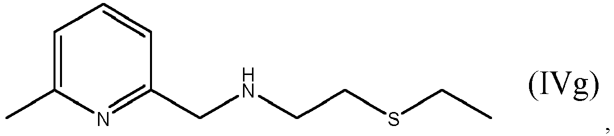 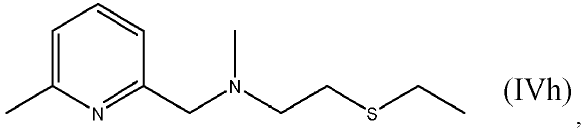 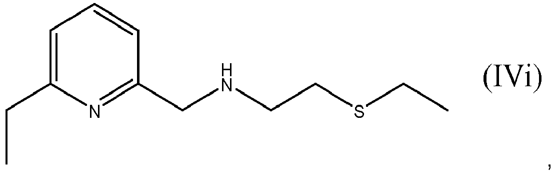 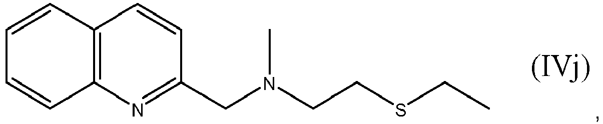 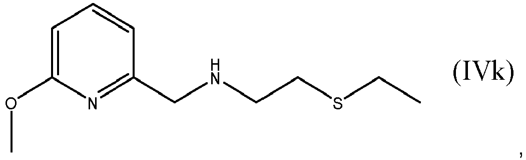 and 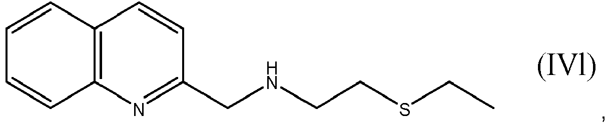 are used.

Therefore the present invention relates to a process (P2), which is process (P), (P1"), (P1"') or (P1""), wherein the following catalysts of formula (III)

[M(L)(X)ₐ(L')_{b}] **(III)**,

wherein
M is a transition metal chosen from the group consisting of Os, Co, Ru and Fe, and
X is a halogen anion, a carboxylate (such as acetate or benzoate), borohydride (such as BH₄⁻), hydride, BF₄⁻ or PF₆⁻, and
L' is a monodentate phosphine ligand, and
L is a tridentate ligand of formula (IV) 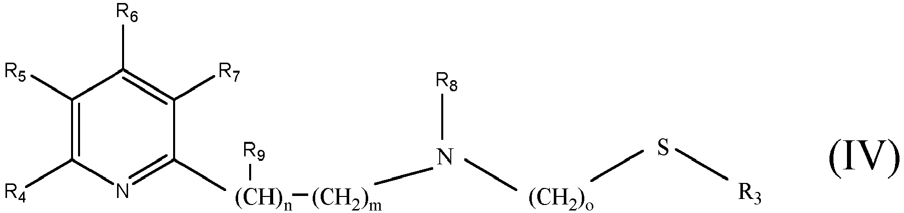 wherein
   R₃ is -CH₃ or -CH₂CH₃, and
   R₄ is H; -CH₃; -CH₂CH₃; -OCH₃ or -OCH₂CH₃, and
   R₅ is H; -CH₃; -CH₂CH₃; -OCH₃ or -OCH₂CH₃, and
   or R₄ and R₅ form a C₄-C₈ ring system, which can be aliphatic or aromatic, and
   R₆ is H; -CH₃; -CH₂CH₃; -OCH₃ or -OCH₂CH₃, and
   R₇ is H; -CH₃; -CH₂CH₃; -OCH₃ or -OCH₂CH₃, and
   R₈ is H; -CH₃ or -CH₂CH₃, and
   R₉ is -CH₃ or -CH₂CH₃, and
   m is 0, 1 or 2, and
   n is 0, 1 or 2,
   with the proviso that the sum of m+n is 1 or 2,
   o is 2 or 3,
   a is 0, 1, 2, or 3,
   b is 0, 1, 2, or 3,
   with the proviso that the sum of a+b is 2 or 3,
   are used.

Therefore the present invention relates to a process (P2'), which is process (P), (P1"), (P1'") or (P1""), wherein the following catalysts of formula (III)

[M(L)(X)ₐ(L')_{b}] **(III)**,

wherein
M is a transition metal chosen from the group consisting of Ru and Fe, and
X is a halogen anion (preferably Cl⁻), and
L' is triphenylphosphine, and
L is a tridentate ligand of formula (IV) 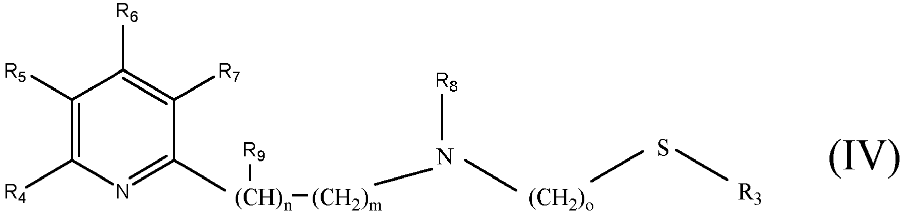 wherein
   R₃ is -CH₃ or -CH₂CH₃, and
   R₄ is H; -CH₃; -CH₂CH₃; -OCH₃ or -OCH₂CH₃, and
   R₅ is H or -CH₃, and
   or R₄ and R₅ form a C₄-C₈ ring system, which can be aliphatic or aromatic, and
   R₆ is H or -CH₃, and
   R₇ is H or -CH₃, and
   R₈ is H or -CH₃, and
   R₉ is -CH₃, and
   m is 0 or 1, and
   n is 0 or 1,
   with the proviso that the sum of m+n is 1,
   o is 2,
   a is 1 or 2,
   b is 1 or 2,
   with the proviso that the sum of a+b is 3,
   are used.

Therefore the present invention relates to a process (P2"), which is process (P), (P1"), (P1"') or (P1""), wherein the following catalysts of formula (III')

M(L)(X)₂(L') **(III')**,

wherein
M is Ru or Fe, and
X is Cl⁻, and
L' is PPh₃, and
L is a tridentate ligand chosen from the group consisting of the ligands of formulae (IVa) - (IVI) 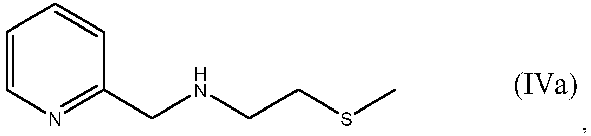 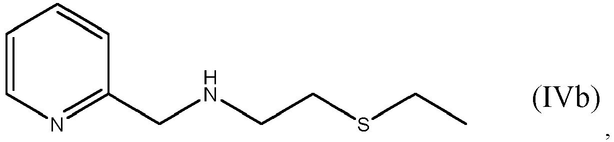 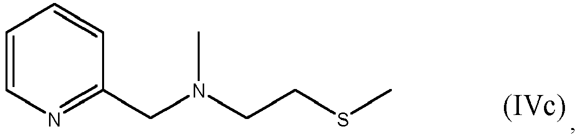 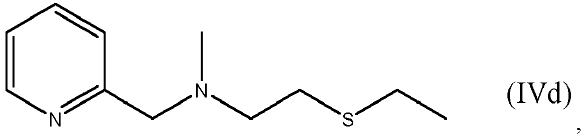 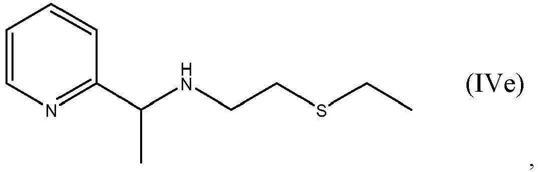 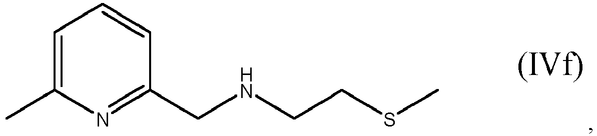 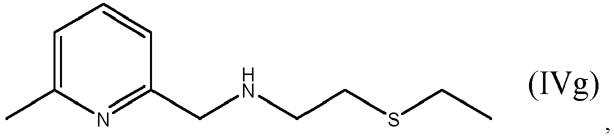 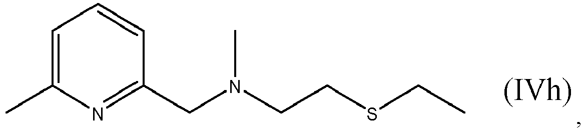 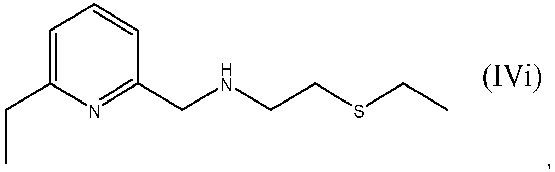 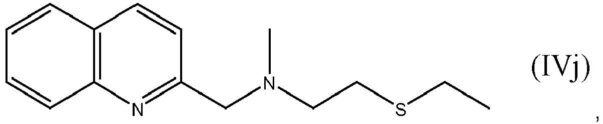 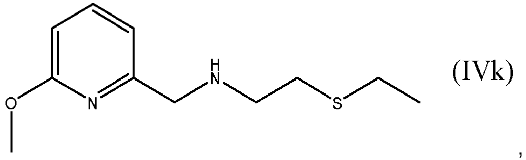 and 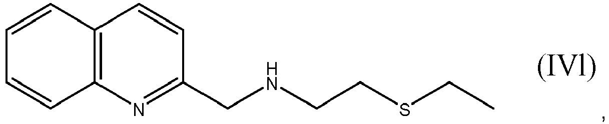 are used.

A preferred embodiment of the present invention also relates to a process (P3), which is process (P), (P1"), (P1'"), (P1""), (P2), (P2') or (P2"), wherein a compound of the formula (IIa) 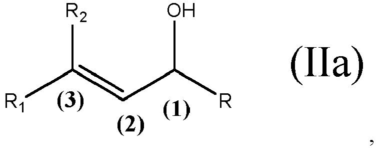 wherein
- R: is H or -CH₃; and
- R₁: is an aromatic ring system which is unsubstituted or an aromatic ring system which is substituted; a heteroaromatic ring system which is unsubstituted or a heteroaromatic ring system which is substituted; an aliphatic ring system which is unsubstituted or an aliphatic ring system which is substituted; a heteroaliphatic ring system which is unsubstituted or a heteroaliphatic ring system which is substituted; and
- R₂: is H or -CH₃,
is produced

Another preferred embodiment of the present invention also relates to a process (P3'), which is process (P3), wherein a compound of the formula (IIa') 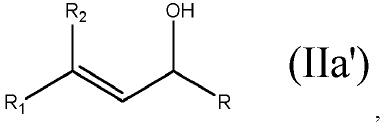 wherein
R is H or -CH₃ and
and
R₁ is benzene ring which is unsubstituted or a benzene ring which is substituted or furan ring which is unsubstituted or a furan ring which is substituted; hexane ring which is unsubstituted or a hexane ring which is substituted; a hexene ring which is unsubstituted or a hexene ring system which is substituted
R₂ is H or -CH₃,
is produced.

Another preferred embodiment of the present invention also relates to a process (P4), which is process (P), (P1"), (P1"'), (P1""), (P2), (P2') or (P2"), wherein a compound of the formula (IIa) 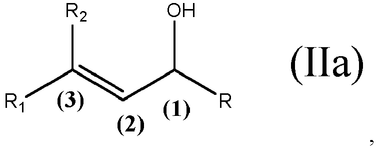 wherein
- R: is H or -CH₃; and
- R₁: is an unsubstituted C₃ - C₈ alkyl group, which can be linear or branched and which can be partially unsaturated or an unsubstituted C₁₂ - C₁₈ alkyl group, which can be linear or branched and which can be partially unsaturated,
- R₂: is H or -CH₃,
is produced.

Another preferred embodiment of the present invention also relates to a process (P5), which is process (P), (P1"), (P1'"), (P1""), (P2), (P2') or (P2"), wherein a compound of the formula (IIa) 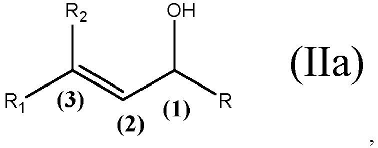 wherein
- R: is H or -CH₃; and
R₁ forms together with the C-C double bond of formula (IIa) a 5 to 8 membered aliphatic ring system, which can be substituted or a 5 to 8 membered aromatic ring system, which can be substituted;
or R₁ forms together with the C-C double bond of formula (IIa) a 5 to 8 membered heteroaliphatic ring system, which can be substituted or a 5 to 8 membered aromatic ring system, which can be substituted
or R₁ forms together with the C-C double bond of formula (IIa) a 5 to 8 membered aromatic ring system, which can be substituted or a 5 to 8 membered aromatic ring system, which can be substituted
or R₁ forms together with the C-C double bond of formula (IIa) a 5 to 8 membered heteroaromatic ring system, which can be substituted or a 5 to 8 membered aromatic ring system, which can be substituted and
- R₂: is H or -CH₃,
is produced.

Another preferred embodiment of the present invention also relates to a process (P5), which is process (P), (P1"), (P1'"), (P1""), (P2), (P2') or (P2"), wherein a compound of the formula (IIa) 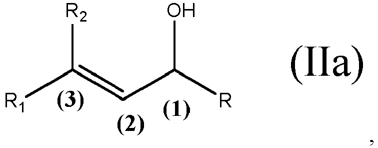 wherein
R forms together with carbon atom (2) a 4 to 8 membered aliphatic ring system, which can be substituted; and and
- R₁: forms together with the C-C double bond of formula (IIa) a 5 to 8 membered aliphatic ring system, which can be substituted or a 5 to 8 membered aromatic ring system, which can be substituted; and
- R₂: is H or -CH₃,
is produced.

Another preferred embodiment of the present invention also relates to a process (P5'), which is process (P5), wherein a compound of the formula (IIa') 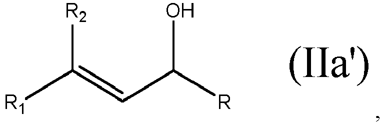 wherein
- R: is H or -CH₃ and
- R₁: is H; benzene ring which is unsubstituted or a benzene ring system which is substituted; and
- R₂: is H or -CH₃,
is produced.

A preferred embodiment of the present invention also relates to a process (P6), which is process (P), (P1"), (P1'"), (P1""), (P2), (P2') or (P2"), wherein any compound of formula (IIa¹) to (IIa¹²) 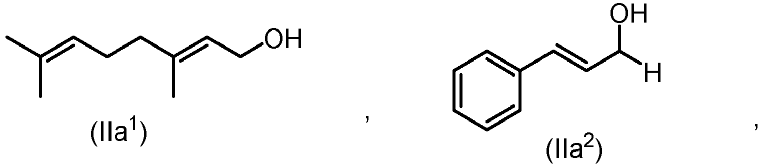 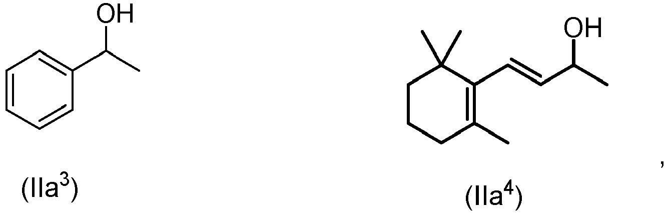 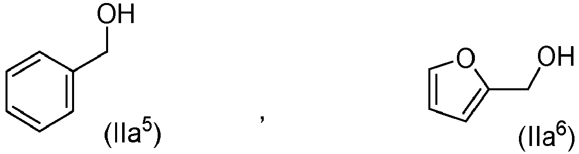 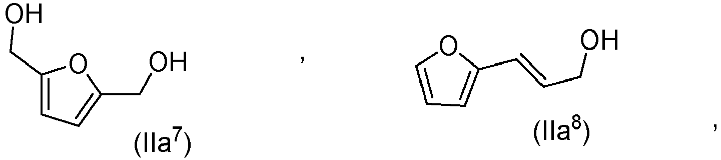 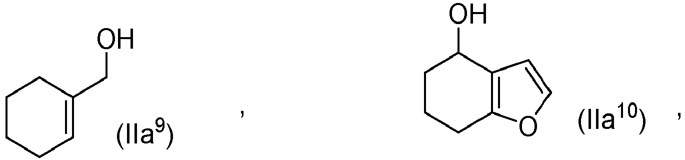 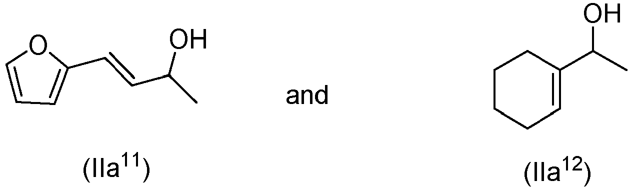 is produced.

Some of the catalysts of the present invention are also new.

Therefore the present invention also relates to a catalyst (C") of formula (III)

[M(L)(X)ₐ(L')_{b}] **(III)**,

wherein
M is a transition metal chosen from the group consisting of Ru and Fe, and
X is a halogen anion (preferably Cl⁻), and
L' is triphenylphosphine, and
L is a tridentate ligand of formula (IV) 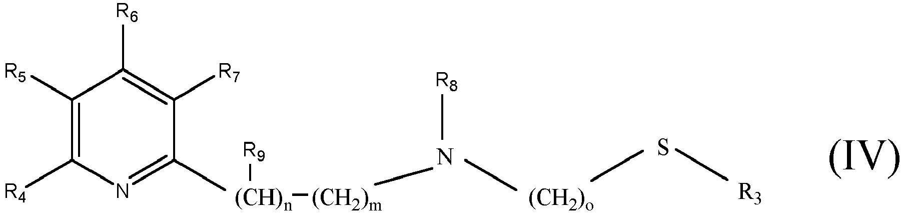 wherein
   R₃ is -CH₃ or -CH₂CH₃, and
   R₄ is H; -CH₃; -CH₂CH₃; -OCH₃ or -OCH₂CH₃, and
   R₅ is H or -CH₃, and
   or R₄ and R₅ form a C₄-C₈ ring system, which can be aliphatic or aromatic, and
   R₆ is H or -CH₃, and
   R₇ is H or -CH₃, and
   R₈ is H or -CH₃, and
   R₉ is -CH₃, and
   m is 0 or 1, and
   n is 0 or 1,
   with the proviso that the sum of m+n is 1,
   o is 2,
   a is 1 or 2,
   b is 1 or 2,
   with the proviso that the sum of a+b is 3.

Therefore the present invention also relates to a catalyst (C'") of formula (III')

M(L)(X)₂(L') **(III')**,

wherein
M is Ru or Fe, and
X is Cl⁻, and
L' is PPh₃, and
L is a tridentate ligand chosen from the group consisting of the ligands of formulae (IVa) - (IVI) 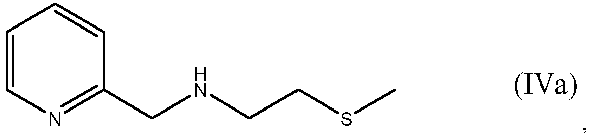 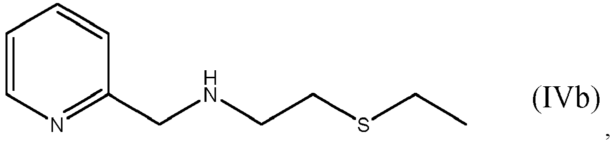 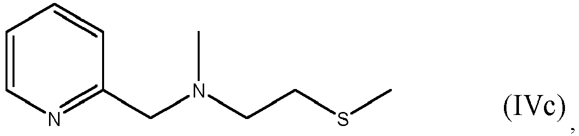 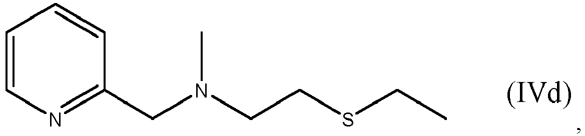 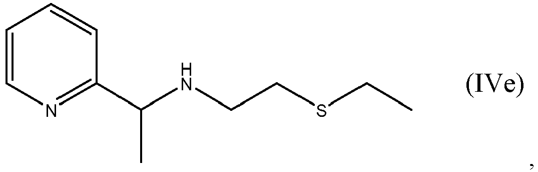 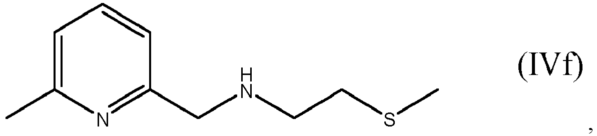 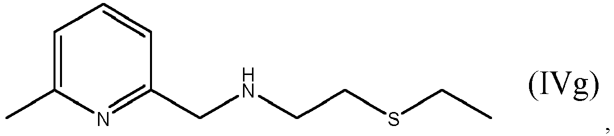 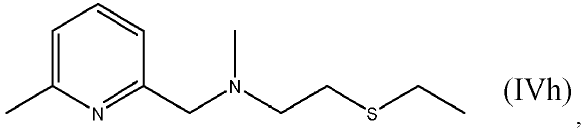 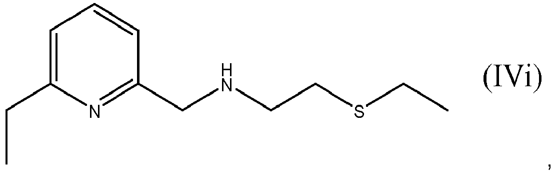 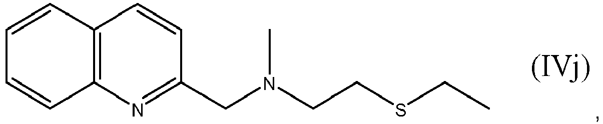 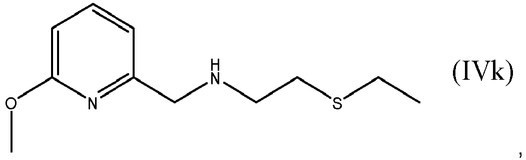 and

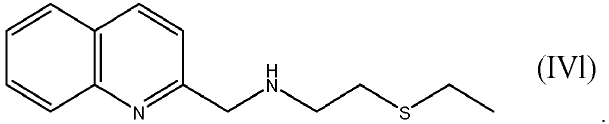

In the following the synthesis of the catalyst used in the selective reduction of the present invention is described.

### Production of the ligand L (compounds of formula (IV))

The ligand (L) is usually made first and this ligand (L) is then used afterwards to synthesise the transition metal based catalyst of formula (III).

The production of the ligands (wherein R₈ is H) is usually done by the following reaction scheme (RS): 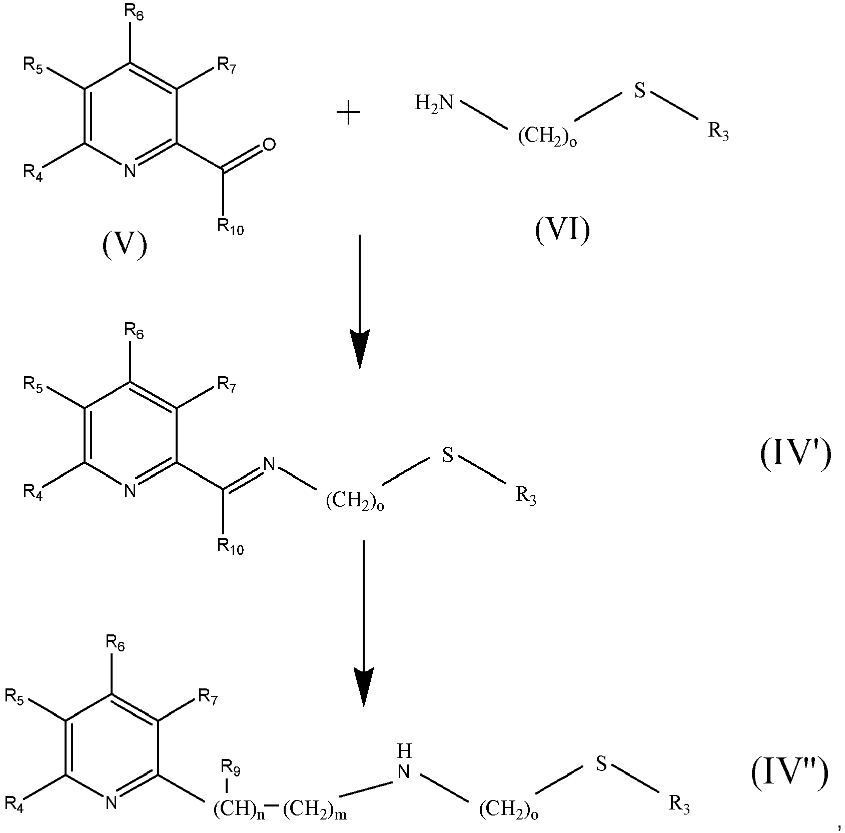 wherein R₁₀ is H or has the same meaning as R₉, all other substituents and o have the meanings as defined above.

To obtain the ligands (wherein R₈ is -CH₃ or -CH₂CH₃), the process of RS is carried out and then in an additional step the amino group is alkylated.

The process of the production of the ligand is usually carried out in a solvent (or a mixture of solvents).

Suitable solvents are esters, ethers, amides, hydrocarbons, halogenated hydrocarbons and alcohols. Preferred solvents are CH₂Cl₂, toluene, ethyl acetate, THF, methanol and ethanol.

The process of the production of the ligand is usually carried out at temperature of between 0 and 120°C (preferably 0 - 40°C).

The process of the production of the ligand is usually carried at ambient pressure.

The obtained ligand of formula (IV") (with R₈ = H) is removed from the reaction mixture by extraction and can be further purified if required. The yield is very good.

To obtain the ligands of formula (IV) wherein R₈ is -CH₃ or -CH₂CH₃, the obtained ligand of formula (IV") is alkylated in an additional step.
This alkylation step can be carried out according to commonly known processes.

### Production of the catalyst (compounds of formula (III))

As stated above some of the catalysts of the present invention are new.

They are produced by commonly known processes. Usually (and preferably in the context of the present invention) they are produced as follows (reaction scheme (RS2)): 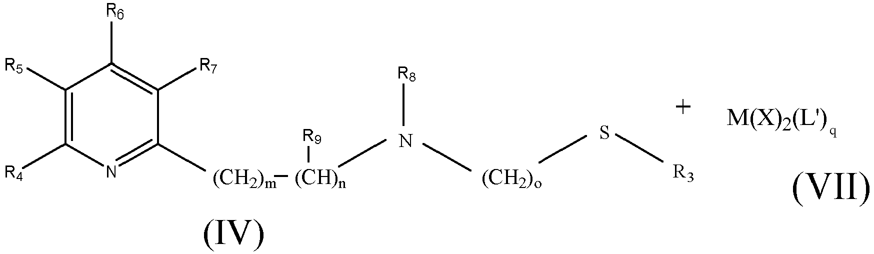 wherein q is 1, 2 or 3 and
all other substituents have the meanings as defined above.

The process to obtain the catalyst (RS2) is usually carried out in a solvent (or a mixture of solvents). Suitable solvents are esters, ethers, amides, hydrocarbons, and alcohols. Preferred solvents are toluene, ethyl acetate, THF and diglyme.

The process to obtain the catalyst is usually carried out at elevated temperature (50 - 180°).

The process to obtain the catalyst is usually carried out at ambient pressure.

The obtained catalyst (in crystalline form) are filtered off and they can be further purified.

As stated above the obtained catalysts are used in the selective reductions (selective hydrogenations), wherein the yield and selectivity of the desired product is excellent.

### Reduction process

The reduction process (selective hydrogenation) of the compound of formula (I) can be carried out according to the following reaction scheme 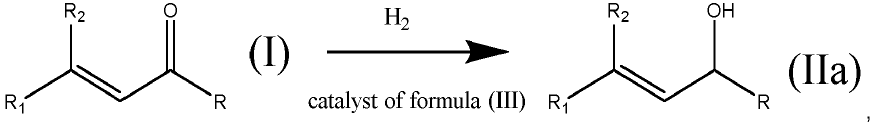 wherein all substituents have meanings as defined above.

In these hydrogenation processes H₂ is added in form of a gas (pure H₂ gas or a mixture).

The catalyst of formula (III) according to the present invention is usually used in an amount of 0.001 - 0.5 mol-% (based on the number of moles of the compounds of formula (I)).

Therefore, the present invention also relates to a process (P7), which is process (P), (P1"), (P1'"), (P1""), (P2), (P2'), (P2"), (P3), (P3'), (P4), (P5), (P5') or (P6), wherein the at least one catalyst of formula (III) is used in an amount of 0.001 - 0.5 mol-% (based on the number of moles of the compounds of formula (I)).

The hydrogenation process can be carried out with (pure) H₂ gas or with a gas which comprises H₂. Preferably the hydrogenation process according to the present invention is carried out with (pure) H₂ gas.

Therefore, the present invention also relates to a process (P8), which is process (P), (P1"), (P1'"), (P1""), (P2), (P2'), (P2"), (P3), (P3'), (P4), (P5), (P5'), (P6) or (P7), wherein the hydrogenation is carried out with (pure) H₂ gas or with a gas which comprises H₂ (preferably with (pure) H₂ gas).

The hydrogenation process can be carried out at ambient pressure as well as at elevated pressure. Preferably the hydrogenation process according to the present invention is carried out at elevated pressure (10 - 50bar), usually in an autoclave (or any other vessel, which can resist the pressure.

Therefore, the present invention also relates to a process (P9), which is process (P), (P1"), (P1'"), (P1""), (P2), (P2'), (P2") (P3), (P3'), (P4), (P5), (P5'), (P6), (P7) or (P8), wherein the hydrogenation is carried out at ambient pressure.

Therefore, the present invention also relates to a process (P10), which is process (P), (P1"), (P1"'), (P1""), (P2), (P2'), (P2") (P3), (P3'), (P4), (P5), (P5'), (P6), (P7) or (P8), wherein the hydrogenation is carried out at out at elevated pressure (10 - 50bar).

The hydrogenation can be carried out in a solvent (or mixture of solvents). Suitable solvents are esters, ethers, amides, hydrocarbons, halogenated hydrocarbons and alcohols. Preferred solvents are CH₂Cl₂, toluene, ethyl acetate, THF, methanol, ethanol and isopropanol, especially preferred solvents are toluene and isopropanol.

Therefore, the present invention also relates to a process (P11), which is process (P), (P1"), (P1"'), (P1""), (P2), (P2'), (P2") (P3), (P3'), (P4), (P5), (P5'), (P6), (P7), (P8), (P9) or (P10), wherein the hydrogenation is carried out in at least one a solvent.

Therefore, the present invention also relates to a process (P11'), which is process (P11), wherein the hydrogenation is carried out in at least one a solvent chosen from the group consisting of esters, ethers, amides, hydrocarbons, halogenated hydrocarbons and alcohols. Preferred solvents are CH₂Cl₂, toluene, ethyl acetate, THF, methanol, ethanol and isopropanol, especially preferred solvents are toluene and isopropanol.

Therefore, the present invention also relates to a process (P11"), which is process (P11), wherein the hydrogenation is carried out in at least one a solvent chosen from the group consisting of CH₂Cl₂, toluene, ethyl acetate, THF, methanol, ethanol and isopropanol (especially preferred are toluene and isopropanol).

The hydrogenation is usually carried out at 30 - 150 °C.

Therefore, the present invention also relates to a process (P12), which is process (P), (P1"), (P1'"), (P1""), (P2), (P2'), (P2") (P3), (P3'), (P4), (P5), (P5'), (P6), (P7), (P8), (P9), (P10), (P11), (P11') or (P11"), wherein the hydrogenation is carried out at 30 - 150 °C.

It is also possible to reduce the compound of formula (I) selectively by a transfer hydrogenation process. In that case no H₂ gas needs to be added. As reductant any suitable hydrogen donor can be used, including secondary alcohols, such as isopropanol and formic acid, its salts or derivatives.

Therefore the present invention also relates to a process (P13), which is process (P), (P1"), (P1'"), (P1""), (P2), (P2'), (P2") (P3), (P3'), (P4), (P5), (P5'), (P6) or (P7), wherein the reduction is a transfer hydrogenation.

The following examples serve to illustrate the invention. If not otherwise stated the temperature is given in °C.

### Examples

### General:

Transition metal precursors, reagent and solvents were obtained from commercial sources and used as received unless noted otherwise. GC analysis was carried out on an Agilent 7890B GC system with a HP-5 normal-phase silica column, using Helium as a carrier gas and dodecane as an internal standard. NMR spectra were recorded on a Bruker AV400, Bruker AV300 or Bruker Fourier300 NMR spectrometer. ¹H and ¹³C-NMR spectra were referenced w.r.t. the solvent signal. Chemical shifts are in ppm, coupling constants in Hz. HR-MS measurements were recorded on an Agilent 6210 Time-of-Flight LC/MS, peaks as listed correspond to the highest abundant peak and are of the expected isotope pattern.

### Ligand synthesis

### Example 1: 2-(ethylthio)-N-((6-methylpyridin-2-yl)methyl)ethan-1-amine [ligand of formula (IVg)]

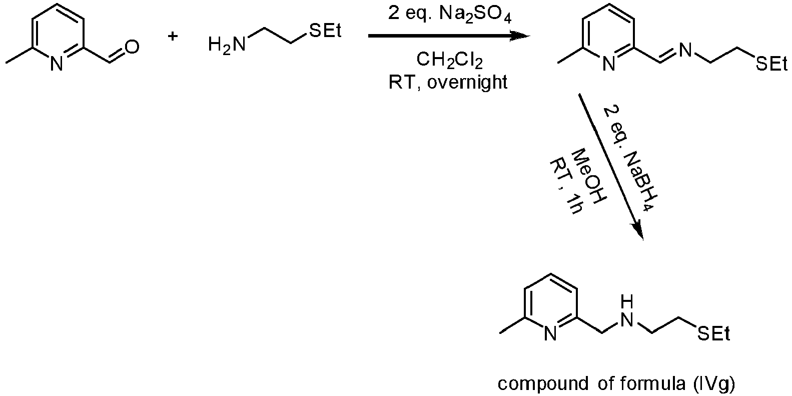

6-methylpyridine-2-carboxaldehyde (3.0 g, 25 mmol) and 2-(Ethylthio)ethylamine (2.63 g, 2.8 mL, 25 mmol) were dissolved in CH₂Cl₂ (75 mL), then Na₂SO₄ (7.1 g, 50 mmol) was added. The suspension was stirred at room temperature overnight, filtered and the filter cake was washed with CH₂Cl₂. The combined volatiles were removed *in vacuo,* yielding 5.45 g of imine as brown oil, which was used directly in the following step without further purification. Therefore, the imine was dissolved in MeOH (50 mL) and NaBH₄ (1.9 g, 51 mmol) was added portionwise at 0 °C. The mixture was stirred at room temperature for another hour, after which the solvent was removed *in vacuo.* Then CH₂Cl₂ (20 mL) and water (20 mL) were added. The aqueous layer was extracted with CH₂Cl₂ (three times 20 mL). The combined organic layers were washed with brine (20 mL) and dried over Na₂SO₄. Evaporating the solvent and drying *in vacuo* yielded 4.95 g (94%) of the ligand of formula (IVg) as an orange oil, which was directly used for complex synthesis.
¹H-NMR (300 MHz, CDCl₃): δ 7.45 (t, 1H, *J* = 7.6, CHₐᵣₒₘ), 7.07 (d, 1H, *J* = 7.8, CHₐ₋ᵣₒₘ), 6.96 (d, 1H, *J* = 7.5, CHₐᵣₒₘ), 3.84 (s, 2H), 2.80 (dt, 2H), 2.66 (dt, 2H), 2.48 (m, 5H), 1.23 (t, 3H, *J* = 7.4) ppm.
¹³C-NMR (75 MHz, CDCl₃): δ 158.9, 157.8, 136.5, 121.3, 118.9, 54.9, 48.2, 31.8, 25.6, 24.4 ppm.
HRMS (ESI+): calculated for C₁₁H₁₈N₂S: 210.1191; found 211.1265 (M+H), 233.1082 (M+Na).

### Example 2: 2-(methylthio)-N-((pyridin-2-yl)methyl)ethan-1-amine [ligand of formula (IVa)]

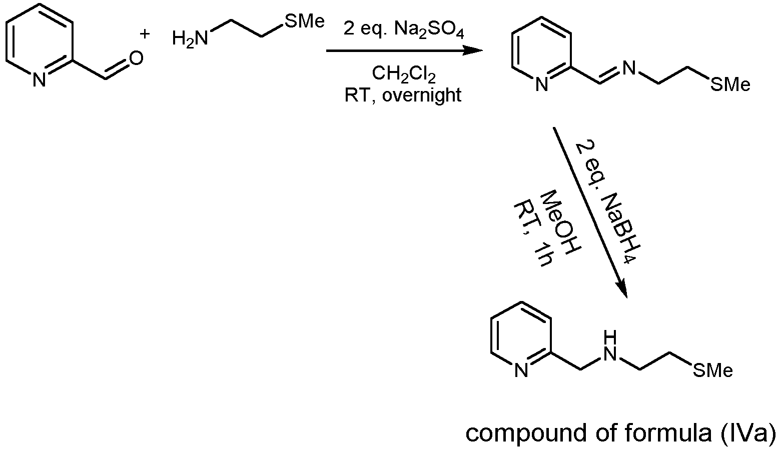

The ligand of formula (IVa) was prepared in analogy to Example 1.
¹H NMR (300 MHz, CD₂Cl₂) δ 8.43 (ddd, 1H, *J* = 4.9 Hz, *J* = 1.8 Hz, *J* = 0.9 Hz, CHₐᵣₒₘ), 7.57 (td, 1H, *J* = 7.7 Hz, *J* = 1.8 Hz, CHₐᵣₒₘ), 7.24 (d, 1H, *J* = 7.8 Hz, CHₐᵣₒₘ), 7.07 (dd, 1H, *J* = 7.5 Hz, *J* = 5.0.7 Hz, CHₐᵣₒₘ), 3.81 (s, 2H), 2.75 (td, 2H, *J* = 6.5 Hz, *J* = 0.8 Hz, CH₂), 2.58 (td, 2H, *J* = 6.5 Hz, *J* = 0.6 Hz, CH₂), 1.99 (s, 3H, CH₃) ppm. ¹³C NMR (75 MHz, CD₂Cl₂): δ 160.2, 149.1, 136.2, 121.9, 121.7, 54.8, 47.6, 34.4, 15.0 ppm.
HRMS (ESI+): calculated for C₉H₁₄N₂S: 182.0878 (M+H): 183.0950; found 183.0950 (M+H).

### Example 3: 2-(ethylthio)-N-((pyridin-2-yl)methyl)ethan-1-amine [ligand of formula (IVb)]

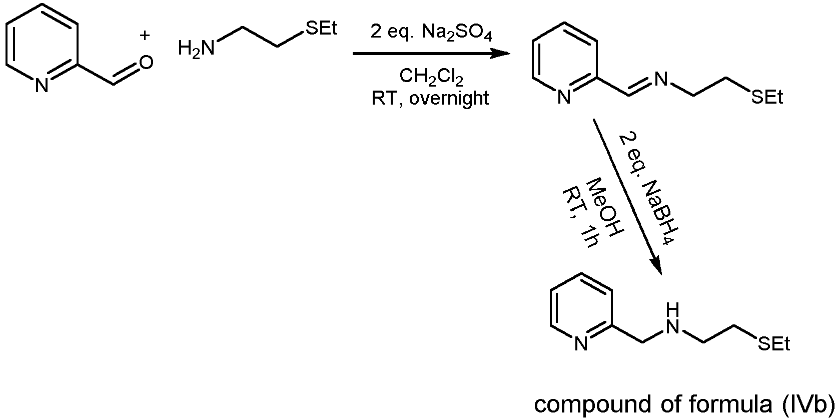

The ligand of formula (IVb) was prepared according to Example 1.
¹H NMR (300 MHz, CD₂Cl₂): δ 8.51 (ddd, 1H, *J* = 4,8 Hz, *J* = 1.5 Hz, J = 0.9 Hz, CHₐᵣₒₘ), 7.64 (td, 1H, *J* = 7.5 Hz, *J* = 1.8 Hz, CHₐᵣₒₘ), 7.32 (d, 1H, *J* = 7,8 Hz, CHₐᵣₒₘ), 7.19 - 7.12 (m, 1H, CHₐᵣₒₘ), 3.88 (s, 2H, CH₂), 2.85 - 2.79 (m, 2H, CH₂), 2.72-2.66 (m, 2H, CH₂), 2.52 (q, 2H, *J* = 7.5 Hz, CH₂), 2.09 (d, 1H, *J* = 9.6 Hz, NH), 1.23 (t, 3H, *J* = 7.4 Hz, CH₃) ppm.
¹³C NMR (75 MHz, CD₂Cl₂): δ 161.6, 149.7, 136.8, 122.5, 122.3, 55.4, 48.9, 32.5, 26.2, 15.3 ppm.
HRMS (ESI+): calculated for C₁₀H₁₆N₂S: 196.1034; (M+H): 197.1107; (M+Na): 219.0926; found 197.1108 (M+H), 219.0929 (M+Na).

### Example 4: 2-(ethylthio)-N-((6-methoxy-pyridin-2-yl)methyl)ethan-1-amine [ligand of formula (lVk)]

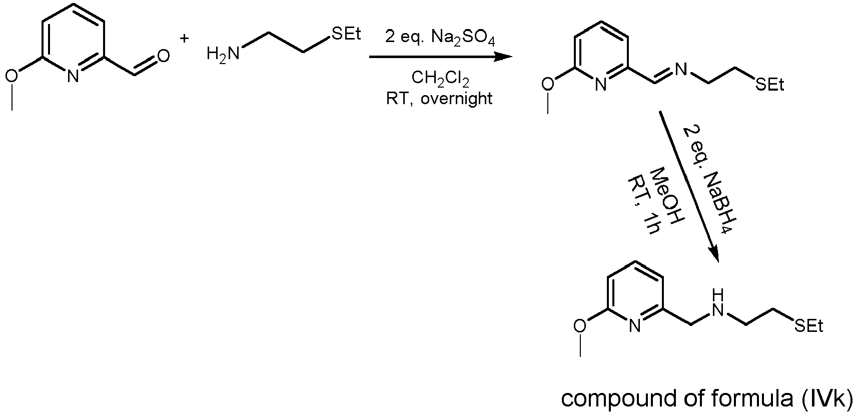

The ligand of formula (IVk) was prepared according to Example 1 in a 84% yield.
¹H-NMR (300 MHz, CDCl₃): δ 7.54 (dd, 1H, *J* = 8.1, *J* = 7.4, CHₐᵣₒₘ), 6.87 (d, 1H, *J* = 7,2), 6.63 (d, 1H, *J* = 8.1), 4.55 (s, NH), 3.92 (s, 3H), 3.90 (m, NH), 3.80 (s, 2H), 2.83 (t, 2H, *J* = 6.5), 2.66 (t, 2H, *J* = 6.5), 2.52 (t, 2H, *J* = 7.5), 1.23 (t, 3H, *J* = 7.2) ppm. ¹³C-NMR (75 MHz, CDCl₃): δ 163.8, 157.3, 138.8, 114.5, 108.7, 54.3, 53.2, 48.1, 32.0, 25.8, 14.8 ppm.
HRMS (ESI+): calculated for C₁₁H₁₈N₂OS: 227.1213 (M+H); found 227.1217 (M+H), 227.1217 (M+Na)

### Example 5: 2-(ethylthio)-N-((quinolin-2-yl)methyl)ethan-1-amine [ligand of formula (IVI)]

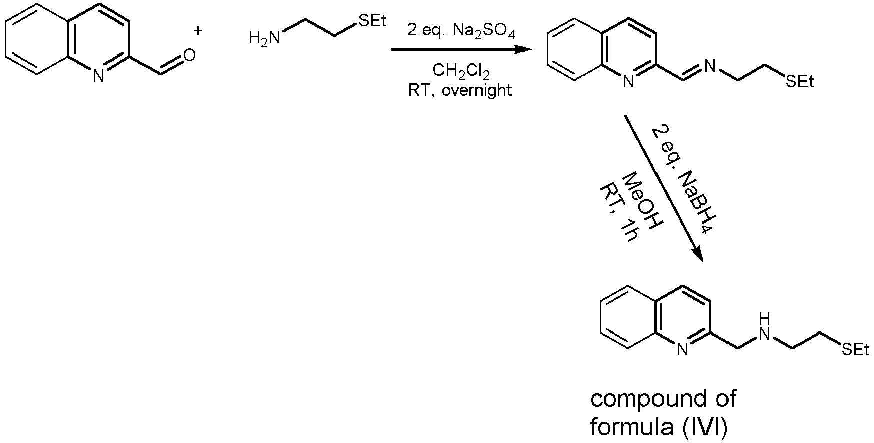

The ligand of formula (IVI) was prepared according to Example 1 and purification by Kugelrohr distillation.
¹H NMR (300 MHz, CD₂Cl₂): δ 8.13 (d, 1H, *J* = 8.4 Hz, CHₐᵣₒₘ), 8.00 (d, 1H, *J* = 8.7 Hz, CHₐᵣₒₘ), 7.82 (dd, 1H, *J* = 8.3 Hz, *J* = 1.5 Hz, CHₐᵣₒₘ), 7.69 (ddd, 3H, *J* = 8.5 Hz, *J* = 6.9 Hz, *J* = 1.5 Hz, CHₐᵣₒₘ), 7.55 - 7.45 (m, 2H, CHₐᵣₒₘ), 4.08 (s, 2H, CH2), 2.89 (td, 2H, *J* = 6.8 Hz, *J* = 1.2 Hz, CH₂), 2.73 (td, 2H, *J* = 6.4 Hz, *J* = 0.9 Hz, CH₂), 2.55 (q, 2H, *J* = 7.4 Hz, CH₂), 2.14 (d, 1H, *J* = 11.4 Hz, NH), 1.24 (t, 3H, J = 7.4 Hz, CH₃) ppm.
¹³C NMR (75 MHz, CD₂Cl₂): δ 161.5, 136.7, 129.8, 129.5, 128.1, 127.9, 126.5, 121.0, 56.0, 49.1, 32.6, 26.2, 15.29 ppm.
HRMS (ESI+): calculated for C₁₄H₁₈N₂S: 246.1191; (M+H): 247.1264; found 247.1267 (M+H)

### Example 6: 2-(ethylthio)-N-(1-(pyridin-2-yl)ethyl)ethan-1-amine [ligand of formula (IVe)]

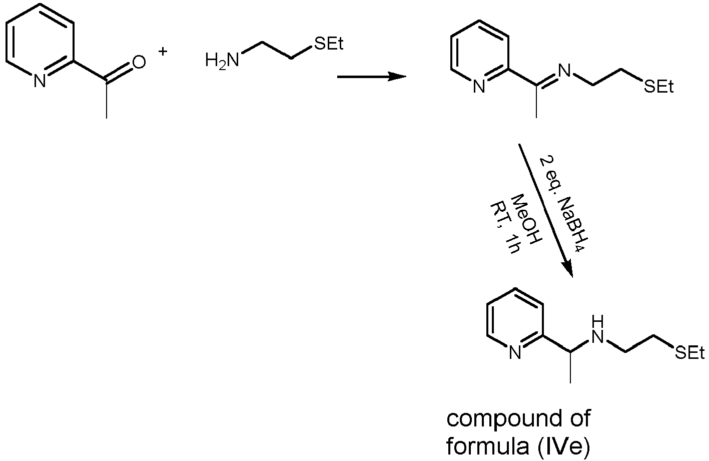

The ligand of formula (IVe) was prepared according to Example 1 with imine formation performed in the presence of 5 mol% of p-toluenesulfonic acid in toluene under reflux conditions and purification by Kugelrohr distillation.
¹H NMR (300 MHz, CD₂Cl₂): δ 8.51 (ddd, 1H, *J* = 4.8 Hz, *J* = 1.9 Hz, *J* = 1.0 Hz, CHₐᵣₒₘ), 7.64 (td, 1H, *J* = 7.6 Hz, *J* = 1.8 Hz, CHₐᵣₒₘ), 7.32 (dt, 1H, *J* = 7.8 Hz, *J* = 1.1 Hz, CHₐᵣₒₘ), 7.14 (ddt, 1H, *J* = 7.5 Hz, *J* = 4.8 Hz, *J* = 1.2 Hz, CHₐᵣₒₘ), 3.84 (q, 1H, *J* = 6.9 Hz, CH), 2.71 - 2.55 (m, 4H, CH₂), 2.47 (q, 2H, *J* = 7.4 Hz, CH₂), 2.05 (d, 1H, *J* = 39.3 Hz, NH), 1.34 (d, 3H, *J* = 6.9 Hz, CH₃), 1.20 (d, 3H, *J* = 7.5 Hz, CH₃) ppm.
¹³C NMR (75 MHz, CD₂Cl₂): δ 165.4, 149.7, 136.9, 122.3, 121.4, 59.7, 47.1, 32.7, 26.1, 23.2, 15.2 ppm.
HRMS (ESI+): calculated for C₁₁H₁₈N₂S: 210.1191; (M+H), 211.1264; (M+Na): 233.1083; found 211.1265 (M+H), 233.1083 (M+Na).

### Example 7: 2-(ethylthio)-N-methyl-N-(pyridin-2-ylmethyl)ethan-1-amine [ligand of formula (IVd)]

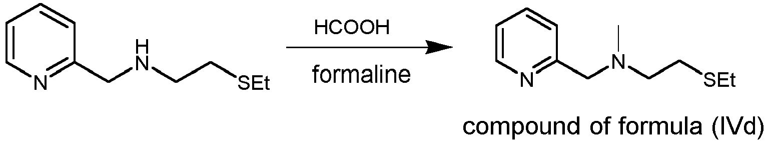

2-(Ethylthio)-N-(pyridin-2-ylmethyl)ethan-1-amine (ligand of formula (IVb), 850 mg, 3.75 mmol), formalin (4 mL of 37% wt formaldehyde in water) and formic acid (4 mL) were stirred at 70°C overnight. All volatiles were removed *in vacuo* and CH₂Cl₂ (10 mL) and saturated NaHCO₃ solution (10 mL) were added. The aqueous layer was extracted with CH₂Cl₂ (three times 10 mL). The combined organic layers were washed with brine (20 mL) and dried over Na₂SO₄. Removal of the solvent yielded 754 mg (3.59 mmol, 96%) of 2-(ethylthio)-*N*-methyl-*N*-(pyridin-2-ylmethyl)ethan-1-amine as an orange liquid (ρ = 1.081 g cm⁻³). The ligand of formula (IVb) was further purified by Kugelrohr distillation.
¹H-NMR (300 MHz, CDCl₃): δ 8.46 (d, 1H, *J* = 5.1, CHₐᵣₒₘ), 7.58 (dt, 1H, *J* = 7.8, *J* = 1.8, CHₐᵣₒₘ), 7.38 (d, 1H, *J* = 7.8, CHₐᵣₒₘ), 7.08 (ddd, 1H, *J* = 7.5, *J* = 4.8, *J* = 1.2, CHₐᵣₒₘ), 3.62 (s, 2H), 2.62 (s, 4H), 2.45 (q, 2H, *J* = 7.4), 2.31 (s, 3H, N-CH3), 1.17 (t, 3H, *J* = 7.4) ppm.
¹³C NMR (101 MHz, CDCl₃): δ 159.2, 149.0, 136.4, 123.1, 122.0, 63.6, 57.3, 56.9, 42.4, 31.9, 29.3, 26.1, 14.8 ppm.
HRMS (ESI+): calculated for C₁₁H₁₈N₂S: 210.1191; found 211. 1265 (M+H), 233.1084 (M+Na)

### Catalyst synthesis

### Example 8: Ru(6-MeNNS^{Et})(PPh₃)Cl₂

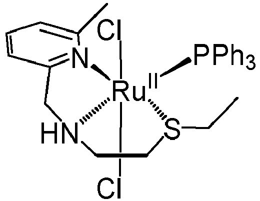

RuCl₂(PPh₃)₃ (1g, 1.04 mmol) and the ligand of formula (IVg) (obtained from Example 1) (231.4 mg, 1.1 mmol) were placed in a 25 mL Schlenk tube under argon atmosphere, and dissolved in dry diglyme (2 mL). The reaction mixture was heated to 165°C for 2 h, allowed to cool down to room temperature and stored at -18°C to precipitate further overnight. Cold Et₂O (2 mL) was added while cooling with a dry ice/*iso*-propanol bath. The precipitate was filtrated by cannula, and washed with Et₂O (5 times 2 mL). The orange powder was dried *in vacuo,* affording 530 mg (79%) of Ru(6-MeNN-S^{Et})(PPh₃)Cl₂ as an orange powder. An equilibrium of two conformations of Ru(6-MeNNS^{Et})(PPh₃)Cl₂ are existent in solution, delivering a doubled set of signals in NMR. For ¹H-NMR only data of the major conformation is given due to overlapping signals.
¹H-NMR (300 MHz, CD₂Cl₂): δ 7.67-7.16 (m, 17H, CHₐᵣₒₘ), 7.01 (d, 1H, *J* = 7.8, CHₐ₋ᵣₒₘ), 5.65 (m, 2H), 4.47 (m, 1H), 3.5 (m, 1H), 3.34 (m, 1H), 3.22 (d, 1H, *J* = 11.1), 2.98 (m, 1H), 2.59 (m, 1H), 1.53 (m, 2H), 0.87 (t, 3H, *J* = 7.5) ppm.
³¹P-NMR (122 MHz, CD₂Cl₂): δ 48.8, 45.8 ppm.
HRMS (ESI+): calculated for C₂₉H₃₂Cl₂N₂PRuS (M+H): 644.0518; found 644.0518 (M+H), 667.0412 (M+Na)

### Example 9: Ru(NNS^{Me})(PPh₃)Cl₂

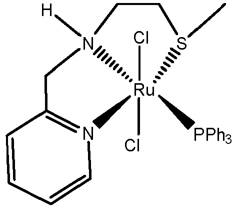

*Ru(NNS^{Me})(PPh₃)Cl₂* was prepared according to Example 8. An equilibrium of two conformations was obtained.
¹H-NMR (300 MHz, CD₂Cl₂): δ 8.47 (d, 1H, *J* = 5.7), 7.72 (m, 1H), 7.56 (m, 6H), 7.32 (m, 10H), 6.86 (t, 1H, *J* = 6.3), 5.45 (s, broad, 1H, NH), 5.20 (t, 1H, *J* = 12.6), 4.38 (m, 1H), 3.41 (m, 2H), 3.26 (d, 1H, *J* = 11.1), 2.55 (m, 1H), 1.50 (s, 3H).
³¹P-NMR (122 MHz, CD₂Cl₂): δ 51.8, 50.7
HRMS (ESI+): calculated for C₂₇H₂₉Cl₂N₂PRuS: 616.0210 (M+); found 616.0197 (M+)

### Example 10: Ru(NNS^{Et})(PPh₃)Cl₂

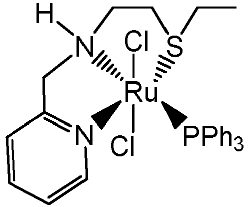

Ru(NNS^{Et})(PPh₃)Cl₂ was prepared according to Example 8. An equilibrium of two conformations was obtained in 84% yield.
¹H-NMR (300 MHz, CD₂Cl₂): δ 8.45 (d, 1H, *J* = 5.7), 7.72 (m, 1H), 7.57 (m, 6H), 7.34 (m, 10H), 6.86 (t, 1H, *J* = 6.3), 5.49 (s, broad, 1H, NH), 5.22 (t, 1H, *J* = 13.5), 4.40 (m, 1H), 3.47 (m, 2H), 3.36 (m, 1H), 2.80 (m, 1H), 2.52 (m, 1H), 1.27 (m, 2H), 1.19 (m, 1H), 0.95 (t, 3H, *J* = 7.5)
³¹P-NMR (122 MHz, CD₂Cl₂): δ 51.8, 50.7
HRMS (ESI+): calculated for C₂₈H₃₁Cl₂N₂PRuS: 630.0366 (M+); found 630.0388 (M+), 653.0270 (M+Na)

### Example 11: Ru(6-MeONNS^{Et})(PPh₃)Cl₂

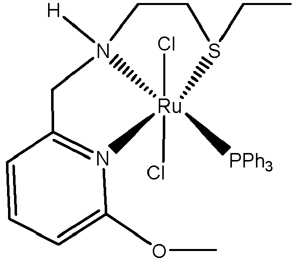

Ru(6-MeONNS^{Et})(PPh₃)Cl₂ was prepared according to Example 8. An equilibrium of two conformations was obtained in 88% yield.
¹H-NMR (400 MHz, CD₂Cl₂): δ 7.94 (m, 2H), 7.65 (m, 2H), 7.42-7.14 (m, 12H), 7.07 (d, 1H, *J* = 7.6), 6.56 (d, 1H, *J* = 8.4), 5.56-5.36 (m, 2H), 4.46 (m, 1H), 3.50-3.19 (m ,2H), 3.21 (dd, 1H, *J* = 11.0, *J* = 2.2), 2. 87 (m, 1H), 2.83 (s, 3H, twinned), 2.50 (m, 1H), 1.33 (m, 1H), 0.87 (t, 3H, twinned, overlapping)
³¹P-NMR (122 MHz, CD₂Cl₂): δ 47.2, 45.9
HRMS (ESI+): calculated for C₂₉H₃₂Cl₂N₂OPRuS (M+H): 660.0468; found: 660.0469 (M+H), 683.0363 (M+Na)

### Example 12: Ru(QuinNS^{Et})(PPh₃)Cl₂

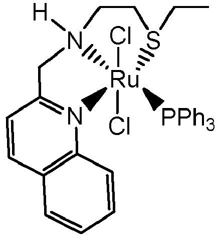

Ru(QuinNS^{Et})(PPh₃)Cl₂ was prepared according to Example 8. An equilibrium of two conformations was obtained.
¹H-NMR (300 MHz, CD₂Cl₂): δ 8.12 (d, 2H, *J* = 8.4), 7.74-6.66 (m, 19H), 5.90 (s, broad, NH), 5.74 (t, 1H, *J* = 13.3), 4.72 (m, 1H), 3.58-3.40 (m, 3H), 3.05 (m, 1H), 2.72 (m, 1H), 1.66 (m, 1H), 0.95 (t, 3H, *J* = 7.5)
³¹P NMR (122 MHz, CD₂Cl₂): δ 48.90, 45.86
HRMS (ESI+): calcd. for C₃₂H₃₃Cl₂N₂PRuS: 680.0519 (M+); found 680.0500 (M+)

### Example 13: Ru(N-Me-NS^{Et})(PPh₃)Cl₂

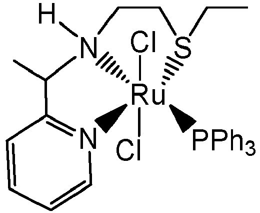

Ru(N-Me-NS^{Et})(PPh₃)Cl₂ was prepared according to Example 8. An equilibrium of two conformations was obtained.
¹H-NMR (300 MHz, CD₂Cl₂): δ 8.53 (d, 1H, *J* = 5.7), 7.72 (m, 1H), 7.57 (m, 6H), 7.33 (m, 10H), 6.85 (t, 1H, *J* = 6.6), 5.35 (m, 1H), 4.93 (s, broad, NH), 3.68-3.31 (m, 3H), 2.81 (m, 1H), 2.53 (m, 1H), 1.80 (d, 3H, *J* = 6.9), 1.25 (m, 1H), 0.97 (t, 3H, *J* = 7.2) ³¹P NMR (122 MHz, CD₂Cl₂): δ 51.5, 50.3
HRMS (ESI+): calculated for C₂₉H₃₃Cl₂N₂PRuS: 644.0518 (M+); found 644.0513 (M+)

### Example 14: Ru(NN^{Me}S^{Et})(PPh₃)Cl₂

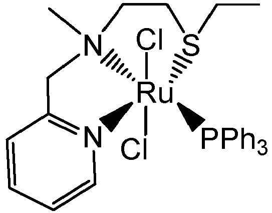

Ru(NN^{Me}S^{Et})(PPh₃)Cl₂ was prepared according to Example 8 . An equilibrium of two conformations was obtained in 54%.
¹H-NMR (300 MHz, CD₂Cl₂): δ 8.11 (d, 1H, *J* = 5.7), 7.92 (m, 6H), 7.47 (dt, 1H, *J* = 7.5, *J* = 1.5), 7.30 (m, 10H), 6.56 (t, 1H, *J* = 7.5), 5.67 (d, 1H, *J* = 14.4), 3.87 (d, 1H, *J* = 14.4), 3.15 (s, 3H), 2.86 (m, 1H), 2.70 (m, 1H), 2.30 (m, 2H), 0.74 (m, 1H), 0.67 (t, 3H, *J* = 6.9), 0.42 (m, 1 H)
³¹P-NMR (122 MHz, CD₂Cl₂): δ 51.4, 50.4
HRMS (ESI+): calculated for C₂₉H₃₃Cl₂N₂PRuS: 644.0518 (M+); found 644.0505 (M+)

### HYDROGENATION REACTIONS

### Example 15: selective hydrogenation of cinnamaldehyde

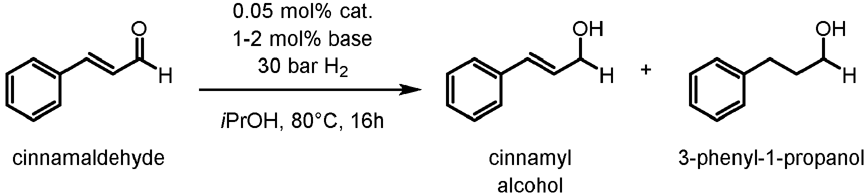

4 mL glass reaction vials and stirring bars were dried overnight at 110°C. The reaction vessels were charged with LiO*t*Bu (1 mg, 0.0125 mmol, 1.25 mol%), closed with PTFE/rubber septa, placed in a multiple reactor inlet suitable for a pressure vessel, and brought under argon atmosphere by three vacuum-argon cycles. With a syringe Ru(NNS^{Et})(PPh₃)Cl₂ (catalyst of Example 10) was added as stock solution in *i*PrOH (1 mL, 0.0005 mol/L, 0.05 mol%), followed by a solution of cinnamaldehyde in *i*PrOH (1 mL, 1 mol/L, 1 mmol). The reaction mixtures were transferred to an argon-filled pressure vessel, which was immediately flushed with three nitrogen and three hydrogen cycles, then pressurized to 30 bar hydrogen, heated to 80°C and stirred for 16h. After that, the pressure vessel was allowed to cool down to room temperature and depressurized. The reaction mixtures were filtered over silica and rinsed with ethanol (2 mL) after which dodecane was added as an internal standard prior to GC analysis. The products are determined based on GC analysis retention time. The given values for conversion (C), yield (Y), and selectivity (S) [%] are mol% with regard to the initial cinnamaldehyde amount, and corrected by dodecane.
The results are summarized in Table 1

The same conditions were chosen for the hydrogenation wherein the catalyst of example 9 (*Ru(NNS^{Me})(PPh₃)Cl₂ was* used. These results are summarized in Table 2

**Table 1:**

| **Exp** | **Base** | **Conversion** | **Cinnamylalcohol** | | **3-phenyl-1-propanol** | |
|---|---|---|---|---|---|---|
| | **1-2 mol%** | **C [%]** | **Y [%]** | **S [%]** | **Y [%]** | **S [%]** |
| **15a** | - | 19 | 0 | 0 | 0 | 0 |
| **15b** | LiO*t*Bu | 96 | 72 | 75 | 4 | 4 |
| **15c** | KO*t*Bu | 99 | 84 | 85 | 5 | 5 |
| **15d** | CaCO₃ | 16 | 2 | 13 | 0 | 0 |
| **15e** | Na(COOPh) | 15 | 0 | 0 | 0 | 0 |

**Table 2:**

| **Exp** | **Base** | **Conversion** | **Cinnamylalcohol** | | **3-phenyl-1-propanol** | |
|---|---|---|---|---|---|---|
| | **1-2 mol%** | **C [%]** | **Y [%]** | **S [%]** | **Y [%]** | **S [%]** |
| **15f** | - | 19 | 0 | 0 | 0 | 0 |
| **15g** | LiO*t*Bu | >99 | 78 | 78 | 3 | 3 |
| **15h** | KO*t*Bu | >99 | 69 | 69 | 3 | 3 |
| **15i** | K₂CO₃ | 52 | 23 | 43 | 0 | 0 |

### Example 16:

4 mL glass reaction vials and stirring bars were dried overnight at 110°C. The reaction vessels were charged with LiO*t*Bu (1 mg, 0.0125 mmol, 1.25 mol%), closed with PTFE/rubber septa, placed in a multiple reactor inlet suitable for a pressure vessel, and brought under argon atmosphere by three vacuum-argon cycles. With a syringe the reaction vessels were charged with the catalyst as stock solution in *i*PrOH (1 mL, 0.0005 mol/L, 0.05 mol%), followed by a solution of cinnamaldehyde in *i*PrOH (1 mL, 1 mol/L). The reaction mixtures were transferred to an argon-filled pressure vessel, which was immediately flushed with three nitrogen and three hydrogen cycles, then pressurized to 30 bar hydrogen, heated to 80°C and stirred for 16h. After that, the pressure vessel was allowed to cool down to room temperature and depressurized. The reaction mixtures were filtered over silica and rinsed with ethanol (2 mL) after which dodecane was added as an internal standard prior to GC analysis. The products are determined based on GC analysis retention time. The given values for conversion (C), yield (Y), and selectivity (S) [%] are mol% with regard to the initial cinnamaldehyde amount, and corrected by dodecane.

Catalyst screening experiments with KO*t*Bu were performed accordingly.

**Table 3:**

| **Exp.** | **Cat.** | **Base** | **Conversion** | **Cinnamylalcohol** | | **3-phenyl-1-propanol** | |
|---|---|---|---|---|---|---|---|
| | **0.05 mol%** | **1-2 mol%** | **C [%]** | **Y [%]** | **S [%]** | **C [%]** | **Y [%]** |
| **16a** | Cat of Exp.9 | LiO*t*Bu | 100 | 77 | 77 | 3 | 3 |
| **16b** | Cat of Exp.9 | KO*t*Bu | 100 | 75 | 75 | 4 | 4 |
| **16c** | Cat of Exp.10 | LiO*t*Bu | 100 | 69 | 69 | 8 | 8 |
| **16d** | Cat of Exp.10 | KO*t*Bu | 100 | 67 | 67 | 10 | 10 |
| **16e** | Cat of Exp.8 | KO*t*Bu | 100 | 64 | 64 | 3 | 3 |
| **16f** | Cat of Exp.13 | LiO*t*Bu | 100 | 65 | 65 | 0 | 0 |
| **16g** | Cat of Exp.13 | KO*t*Bu | 100 | 74 | 74 | 5 | 5 |
| **16hi** | Cat of Exp.14 | LiO*t*Bu | 100 | 81 | 81 | 3 | 3 |
| **16i** | Cat of Exp.14 | KO*t*Bu | 100 | 46 | 46 | 2 | 2 |

### Example 17: hydrogenation of different aldehydes/ketones

The compounds of formulae (A), (B) and (C) were hydrogenated.

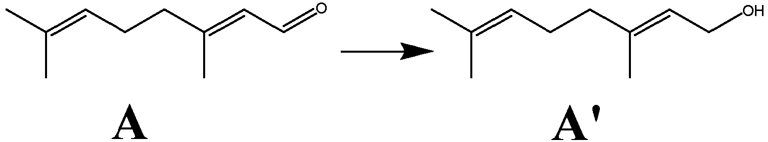

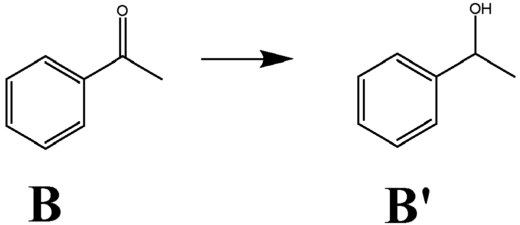

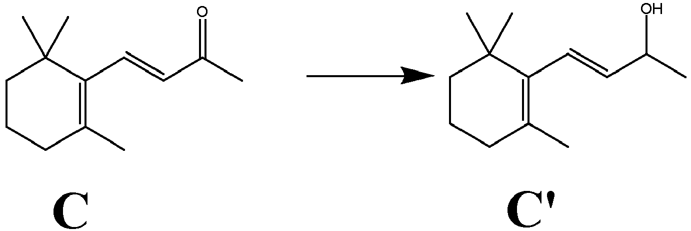

4 mL glass reaction vials and stirring bars were dried overnight at 110°C, closed with PTFE/rubber septa, placed in a multiple reactor inlet suitable for a pressure vessel, and brought under argon atmosphere by three vacuum-argon cycles. With a syringe the reaction vessels were charged with the catalyst as stock solution in *i*PrOH (1 mL, 0.0005 mol/L, 0.05 mol%), followed by a solution of the compound A, B or C in *i*PrOH (1 mL, 1 mol/L, 1 mmol). After that a solution of freshly sublimed the base in THF (12.5 µL, 1 mol/L, 0.0125 mmol, 1.25 mol%) was added with a Hamilton syringe. The reaction mixtures were transferred to an argon-filled pressure vessel, which was immediately flushed with three nitrogen and three hydrogen cycles, then pressurized to 30 bar hydrogen, heated to 80°C and stirred for 16h. After that the pressure vessel was cooled down to room temperature and depressurized. The reaction mixtures were filtered over silica and rinsed with ethanol (2 mL). The products are determined based on GC analysis retention time. The given values [%] are related to GC area%. The results are summarized in the following tables 4a, 4b and 4c.

**Table 4a: hydrogenation of the compound of formula (A)**

| **Exp.** | **Cat.** | **Base** | **Conversion** | **Product Compund A'** | |
|---|---|---|---|---|---|
| | **0.05 mol%** | **1-2 mol%** | **C [%]** | **Y [%]** | **S [%]** |
| **17a** | Cat of Exp.10 | KO*t*Bu | 97 | 97 | 100 |

**Table 4b: hydrogenation of the compound of formula (B)**

| **Exp.** | **Cat.** | **Base** | **Conversion** | **Product Compund B'** | |
|---|---|---|---|---|---|
| | **0.05 mol%** | **1-2 mol%** | **C [%]** | **Y [%]** | **S [%]** |
| **17b** | Cat of Exp.9 | LiO*t*Bu | 100 | 100 | 100 |
| **17c** | Cat of Exp.9 | KO*t*Bu | 100 | 100 | 100 |
| **17d** | Cat of Exp.10 | LiO*t*Bu | 100 | 100 | 100 |
| **17e** | Cat of Exp.10 | KO*t*Bu | 100 | 100 | 100 |

**Table 4c: hydrogenation of the compound of formula (C)**

| **Exp.** | **Cat.** | **Base** | **Conversion** | **Product Compund C'** | |
|---|---|---|---|---|---|
| | **0.05 mol%** | **1-2 mol%** | **C [%]** | **Y [%]** | **S [%]** |
| **17f** | Cat of Exp.9 | LiO*t*Bu | 99 | 96 | 96 |
| **17g** | Cat of Exp.9 | KO*t*Bu | 100 | 96 | 96 |
| **17h** | Cat of Exp.10 | LiO*t*Bu | 99 | 94 | 95 |
| **17i** | Cat of Exp.10 | KO*t*Bu | 99 | 93 | 94 |

### Example 18: Hydrogenation of benzaldehyde

A 100 mL hastelloy autoclave with mechanical stirrer was charged with the catalyst of example 10 (3 mg, 0.005 mmol, 0.05 mol%), benzaldehyde (1.06 g, 10 mmol, 1.01 mL), 20 mL of isopropanol, and freshly sublimed KO*t*Bu (14 mg, 0.125 mmol, 1.25 mol%) under an argon atmosphere. The autoclave vessel was flushed with nitrogen three times, pressurized to 30 bar H₂ and heated to 80°C. After stirring for 1 hour the vessel was allowed to cool down to room temperature and depressurized. The reaction mixture was filtered over SiO₂, and concentrated *in vacuo.* Kugelrohr distillation under vacuum afforded 1.08 g (99% yield) of benzyl alcohol as colourless liquid.

### Example 19: Hydrogenation of furfural

Furfural was hydrogenated according to Example 18 and afforded 0.95 g furfuryl alcohol (99%) as pale yellow liquid.

### Example 20: Hydrogenation of 5-(Hydroxymethyl)furfural

5-(hydroxymethyl)furfural was hydrogenated according to Example 18, with a catalyst amount of 0.5 mol% and a base amount of 5 mol%. The hydrogenation afforded 1.20 g (93%) of 2,5-di(hydroxylmethyl)furan as a white crystalline solid.

### Example 21: Hydrogenation of 3-(2-Furyl)acrolein

3-(2-Furyl)acrolein was hydrogenated according to Example 18 and afforded 1.23 g 3-(2-furyl)-2-propen-1-ol (99% yield) as colourless oil (mixture of isomers).

### Example 22: Hydrogenation of 1-Cyclohexene-1-carboxaldehyde

1-Cyclohexene-1-carboxaldehyde was hydrogenated according to Example 18 and afforded 1.1 g 1-cyclohexene-1-methanol (99% yield) as colourless oil.

### Example 23: Hydrogenation of Cinnamaldehyde

Cinnamaldehyde (F) was hydrogenated according to Example 18 using 25 mmol of substrate, and 50 mL of isopropanol. The resulting yellow oil was purified by column chromatography (SiO2; n-pentane:ethyl acetate 4:1), yielding 3.16 g (94%) of cinnamyl alcohol as white crystals.

### Example 24: Hydrogenation of Perillaldehyde

Perillaldehyde was hydrogenated according to Example 18. The product was isolated by column chromatography (SiO₂; heptane:ethyl acetate 5:1), yielding 1.48 g (96%) of perillyl alcohol as a colourless liquid.
1H NMR (300 MHz, CDCl3): δ 5.63 (broad, 1H), 4.65 (m, 2H), 3.93 (s, 2H), 2.10-1.70 (m, 5H), 1.67 (s, 3H), 1.50 (s, broad, 1H), 1.43 (m, 1H). 13C NMR (75 MHz, CDCl₃): δ 149.8, 137.2, 122.4, 108.6, 67.2, 41.1, 30.4, 27.5, 26.1, 20.8. HRMS (ESI+): calculated for C10H16O: 153.12739 (M+H); found 153.12757 (M+H)+, 175.10946 (M+Na)+.

### Example 25: Hydrogenation of 4,5,6,7-tetrahydro-4-benzofuranone

4,5,6,7-tetrahydro-4-benzofuranone was hydrogenated according to Example 18, affording 1.37 g (99%) of 4,5,6,7-tetrahydro-4-benzofuranol as a colourless liquid.
¹H-NMR (300 MHz, CDCl₃): δ 7.31 (m, 1H), 6.44 (d, *J*_{H-H} = 2.0, 1H), 4.77 (t, *J*_{H-H} = 4.4, 1H), 2.60 (m, 2H), 2.09-1.81 (m, 5H). ¹³C NMR (75 MHz, CDCl₃): δ 152.6, 141.1, 120.0, 109.1, 64.1, 32.7, 23.0, 19.0. HRMS (ESI+): calculated for C₈H₁₀O₂: 139.07536 (M+H); found 139.07548 (M+H), 161.05749 (M+Na).

### Example 26: Hydrogenation of 4-(2-Furanyl)-3-buten-2-one

4-(2-Furanyl)-3-buten-2-one was hydrogenated according to Example 18, affording 1.28 g (93%) of 4-(2-furanyl)-3-buten-2-ol.
1H NMR (300 MHz, CD2Cl2): δ 7.38 (d, JH-H = 1.8, 1H), 6.41 (m, 2H), 6.26 (m, 2H), 4.49 (qd, JH-H = 6.3, 1H), 2.06 (s, broad, 1H), 1.38 (d, JH-H = 6.6, 3H). 13C NMR (75 MHz, CD2Cl2): δ 152.4, 141.9, 132.3, 117.7, 111.3, 108.0, 68.4, 23.4. HRMS (ESI+): calculated for C8H10O2: 161.0573 (M+Na); found 161.05774 (M+Na).

### Example 27: Hydrogenation of 1-(1-Cyclohexen-1-yl)-ethanone

1-(1-Cyclohexen-1-yl)-ethanone was hydrogenated according to Example 18, affording 1.25 g (99%) of α-methyl-1-cyclohexene-1-methanol as a colourless liquid.
¹H NMR (300 MHz, CDCl₃): δ 5.57 (s, broad, 1H), 4.06 (q, *J*_{H-H} = 6.3, 1H), 2.15 (s, 1H), 1.93 (m, 4H), 1.53 (m, 4H), 1.16 (d, *J*_{H-H} = 6.3, 3H). ¹³C NMR (75 MHz, CDCl₃): δ 141.3, 121.3, 72.0, 24.9, 23.6, 22.6, 22.6, 21.5. HRMS (ESI+): calculated for C₈H₁₄O: 149.09369 (M+Na); found 149.09364 (M+Na).

## Claims

1. A process of production of a compound of formula (IIa) 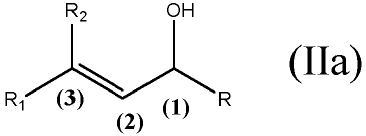 wherein
R is H or a C₁-C₄ alkyl group, or R forms together with carbon atom (2) a 4 to 8 membered aliphatic ring system, which can be substituted; and
R₁ is H; an aromatic ring system which is unsubstituted or an aromatic ring system which is substituted; a heteroaromatic ring system which is unsubstituted or a heteroaromatic ring system which is substituted; an aliphatic ring system which is unsubstituted or an aliphatic ring system which is substituted; a heteroaliphatic ring system which is unsubstituted or a heteroaliphatic ring system which is substituted; -CH₃; -CH₂CH₃; or a C₃ - C₁₀ alkyl group, which can be linear or branched and which can be partially unsaturated
or R₁ forms together with the C-C double bond of formula (IIa) a 5 to 8 membered aliphatic ring system, which can be substituted or a 5 to 8 membered aromatic ring system, which can be substituted;
or R₁ forms together with the C-C double bond of formula (IIa) a 5 to 8 membered heteroaliphatic ring system, which can be substituted or a 5 to 8 membered aromatic ring system, which can be substituted
or R₁ forms together with the C-C double bond of formula (IIa) a 5 to 8 membered aromatic ring system, which can be substituted or a 5 to 8 membered aromatic ring system, which can be substituted
or R₁ forms together with the C-C double bond of formula (IIa) a 5 to 8 membered heteroaromatic ring system, which can be substituted or a 5 to 8 membered aromatic ring system, which can be substituted and
R₂ is H; -CH₃; -CH₂CH₃; or a C₃ - C₁₀ alkyl group, which can be linear or branched and which can be partially unsaturated
by a selective reduction of a compound of formula (I) 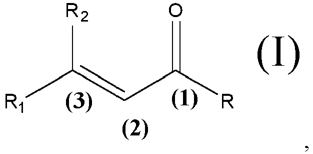 wherein the R, R₁ and R₂ have the same meaning as defined in the compound of formula (IIa),
**characterised in, that** the selective reduction is carried out in the presence of at least one transition metal catalyst of formula (III)
[M(L)(X)ₐ(L')_{b}] (III),
wherein
M is a transition metal and
X is an anion, and
L' is a monodentate ligand, and
L is a tridentate ligand of formula (IV) 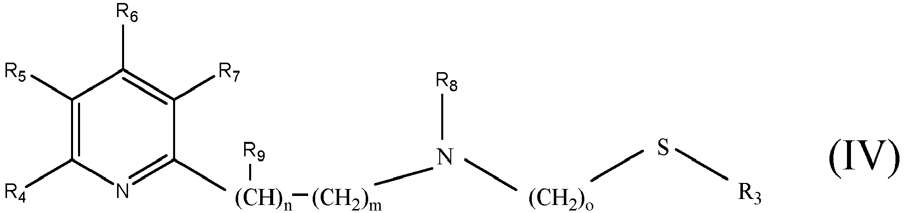 wherein
R₃ is a linear C₁ - C₄ alkyl group, which can be substituted; a branched C₃ - C₄ alkyl group, which can be substituted; or a phenyl group, which can be substituted, and
R₄ is H; a linear C₁ - C₄ alkyl group, which can be substituted; a branched C₃ - C₄ alkyl group, which can be substituted; or OC₁-C₂alkyl, and
R₅ is H; a linear C₁ - C₄ alkyl group, which can be substituted; a branched C₃ - C₄ alkyl group, which can be substituted; or OC₁-C₂alkyl,
or R₄ and R₅ form a C₄-C₈ ring system, which can be aliphatic or aromatic, and
R₆ is H; a linear C₁ - C₄ alkyl group, which can be substituted; a branched C₃ - C₄ alkyl group, which can be substituted;or OC₁-C₂alkyl, and
R₇ is H; a linear C₁ - C₄ alkyl group, which can be substituted; a branched C₃ - C₄ alkyl group, which can be substituted;or OC₁-C₂alkyl, and
R₈ is H or a a linear C₁ - C₄ alkyl group, which can be substituted; a branched C₃ - C₄ alkyl group, which can be substituted, and
R₉ is -CH₃ or -CH₂CH₃, and
m is 0, 1 or 2, and
n is 0, 1 or 2,
with the proviso that the sum of m+n is 1 or 2,
o is 2 or 3,
a is 0, 1, 2, or 3,
b is 0, 1, 2, or 3,
with the proviso that the sum of a+b is 2, 3 or 4,
and wherein the process is carried out in the presence of at least one base of formula (VIII)
M¹(OC₁-C₅alkyl) (VIII),
wherein M¹ is an alkali metal.

2. Process according to claim 1, wherein the process is carried out in the presence of at least one base of formula (VIII'),
M¹(OC₃-C₅alkyl) (VIII')
wherein
M¹ is Li, Na or K.

3. Process according to claim 1 or 2, wherein the process is carried out in the presence of at least one base selected form the group consisting of KOtBu, NaOtBu and LiOtBu.

4. Process according to any one of the preceding claims, wherein
the following catalysts of formula (III)
[M(L)(X)ₐ(L')_{b}] (III),
wherein
M is a transition metal chosen from the group consisting of Os, Co, Ru and Fe, and
X is a halogen anion, a carboxylate (such as acetate or benzoate), borohydride (such as BH4⁻), hydride, BF₄⁻ or PF₆⁻, and
L' is a monodentate phosphine ligand, and
L is a tridentate ligand of formula (IV) 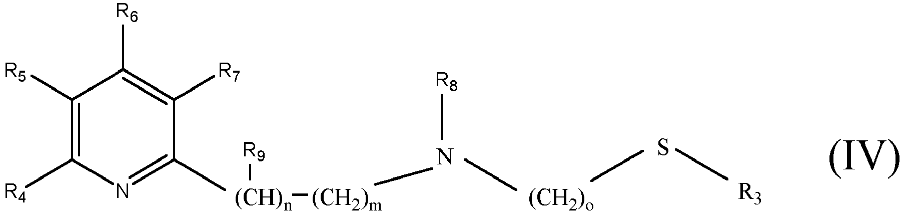 wherein
R₃ is -CH₃ or -CH₂CH₃, and
R₄ is H; -CH₃; -CH₂CH₃; -OCH₃ or -OCH₂CH₃, and
R₅ is H; -CH₃; -CH₂CH₃; -OCH₃ or -OCH₂CH₃, and
R₆ is H; -CH₃; -CH₂CH₃; -OCH₃ or -OCH₂CH₃, and
R₇ is H; -CH₃; -CH₂CH₃; -OCH₃ or -OCH₂CH₃, and
R₈ is H; -CH₃ or -CH₂CH₃, and
R₉ is -CH₃ or -CH₂CH₃, and
or R₄, and R₅ form a C₄-C₈ ring system, which can be aliphatic or aromatic, and
m is 0, 1 or 2, and
n is 0, 1 or 2,
with the proviso that the sum of m+n is 1 or 2,
o is 2 or 3,
a is 0, 1, 2, or 3,
b is 0, 1, 2, or 3,
with the proviso that the sum of a+b is 2 or 3,
are used.

5. Process according to anyone of the preceding claims, wherein the following catalysts of formula (III')
M(L)(X)₂(L') **(III')**,
wherein
M is Ru or Fe, and
X is Cl⁻, and
L' is PPh₃, and
L is a tridentate ligand chosen from the group consisting of the ligands of formulae (IVa) - (IVI) 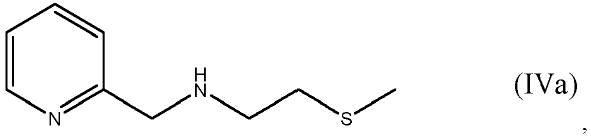 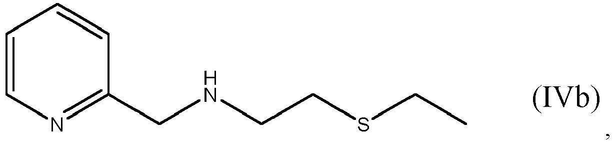 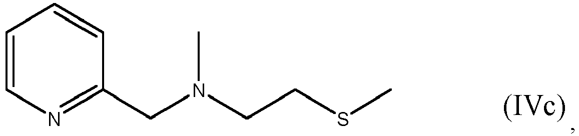 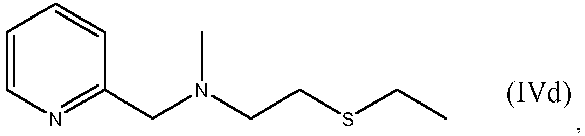 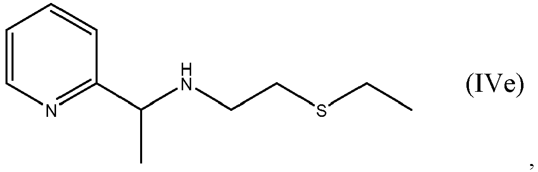 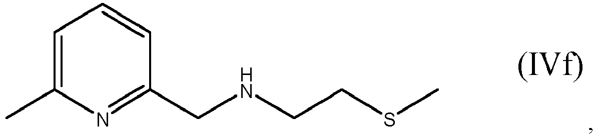 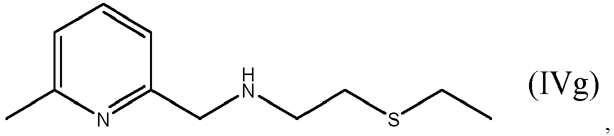 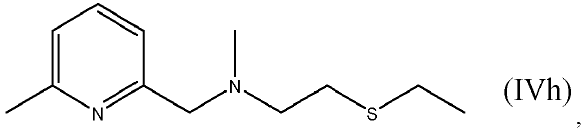 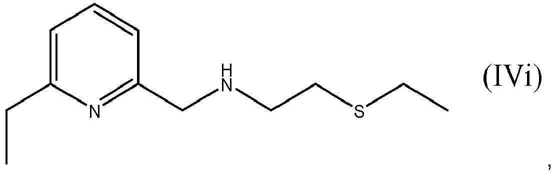 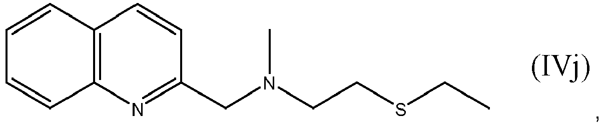 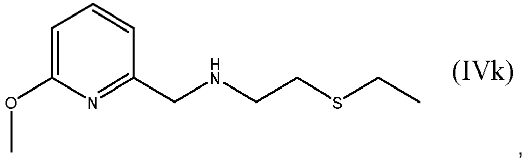 and 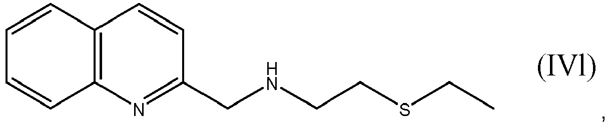 are used.

6. Process according to anyone of the preceding claims, wherein
the catalyst of formula (III) is used in an amount of 0.001 - 0.5 mol-% (based on the number of moles of the compounds of formula (I)).

7. Process according to anyone of the preceding claims, wherein the reduction is a transfer hydrogenation.

8. Process according to anyone of the preceding claims 1 - 6, wherein the process is carried out with H₂ gas.

9. Process according to claim 8, wherein the process is carried out at a pressure of 10 to 50bar.

10. Process according to anyone of the preceding claims, wherein the process is carried out at 30 - 150 °C.

11. A catalyst of formula (III)
[M(L)(X)ₐ(L')_{b}] **(III)**,
wherein
M is a transition metal chosen from the group consisting of Ru and Fe, and
X is a halogen anion (preferably Cl⁻), and
L' is triphenylphosphine, and
L is a tridentate ligand of formula (IV) 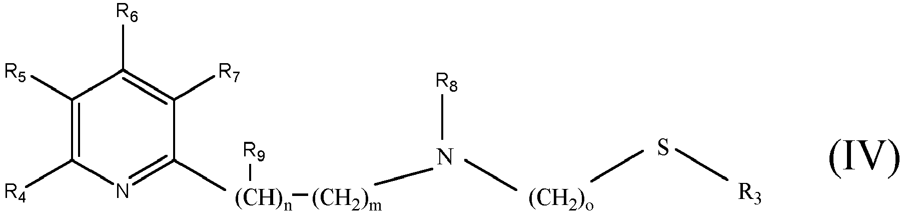 wherein
R₃ is -CH₃ or -CH₂CH₃, and
R₄ is H; -CH₃; -CH₂CH₃; -OCH₃ or -OCH₂CH₃, and
R₅ is H or-CH₃, or R₄ and R₅ form a C₄-C₈ ring system, which can be aliphatic or aromatic, and
R₆ is H or -CH₃, and
R₇ is H or -CH₃, and
R₈ is H or -CH₃, and
R₉ is -CH₃, and
m is 0 or 1, and
n is 0 or 1,
with the proviso that the sum of m+n is 1,
o is 2,
a is 1 or 2,
b is 1 or 2,
with the proviso that the sum of a+b is 3.

12. A catalyst according to claim 11 having formula (III')
M(L)(X)₂(L') **(III')**
wherein
M is Ru or Fe, and
X is Cl⁻, and
L' is PPh₃, and
L is a tridentate ligand chosen from the group consisting of the ligands of formulae (IVa) - (IVI) 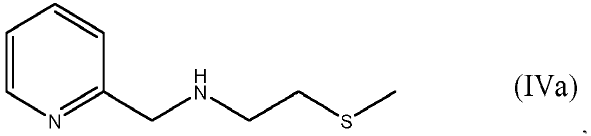 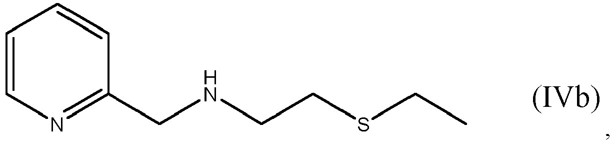 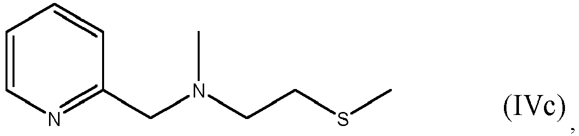 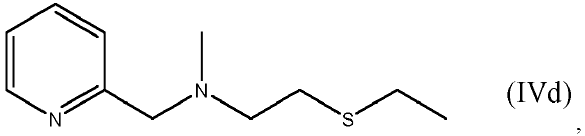 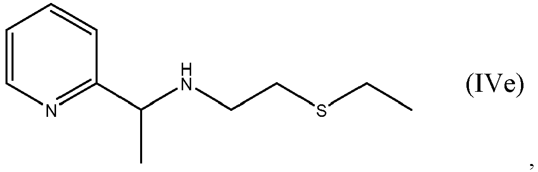 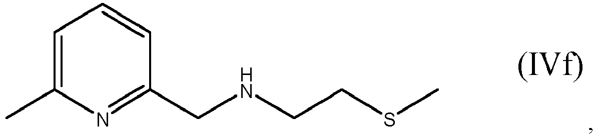 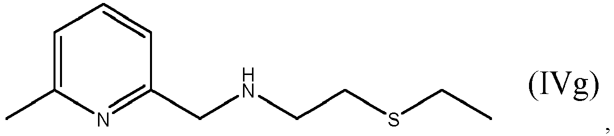 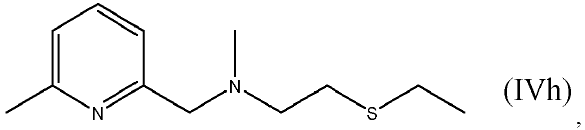 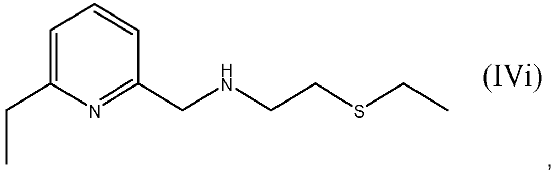 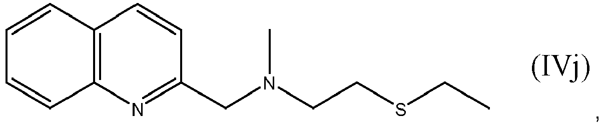 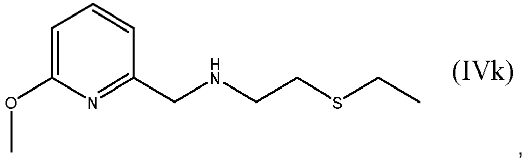 and

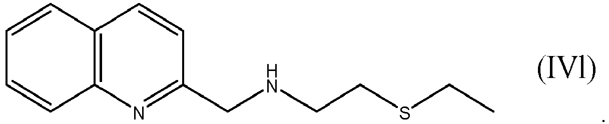

## Patentansprüche

1. Verfahren zur Herstellung einer Verbindung der Formel (IIa) 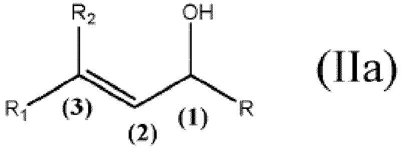 wobei
R für H oder eine C₁-C₄-Alkylgruppe steht oder R zusammen mit Kohlenstoffatom (2) ein 4- bis 8-gliedriges aliphatisches Ringsystem bildet, das substituiert sein kann; und
R₁ für H, ein aromatisches Ringsystem, das unsubstituiert ist, oder ein aromatisches Ringsystem, das substituiert ist, ein heteroaromatisches Ringsystem, das unsubstituiert ist, oder ein heteroaromatisches Ringsystem, das substituiert ist, ein aliphatisches Ringsystem, das unsubstituiert ist, oder ein aliphatisches Ringsystem, das substituiert ist, ein heteroaliphatisches Ringsystem, das unsubstituiert ist, oder ein heteroaliphatisches Ringsystem, das substituiert ist, -CH₃, -CH₂CH₃ oder eine C₃-C₁₀-Alkylgruppe, die linear oder verzweigt sein kann und die teilweise ungesättigt sein kann, steht
oder R₁ zusammen mit der C-C-Doppelbindung von Formel (IIa) ein 5- bis 8-gliedriges aliphatisches Ringsystem, das substituiert sein kann, oder ein 5- bis 8-gliedriges aromatisches Ringsystem, das substituiert sein kann, bildet
oder R₁ zusammen mit der C-C-Doppelbindung von Formel (IIa) ein 5- bis 8-gliedriges heteroaliphatisches Ringsystem, das substituiert sein kann, oder ein 5- bis 8-gliedriges aromatisches Ringsystem, das substituiert sein kann, bildet
oder R₁ zusammen mit der C-C-Doppelbindung von Formel (IIa) ein 5- bis 8-gliedriges aromatisches Ringsystem, das substituiert sein kann, oder ein 5- bis 8-gliedriges aromatisches Ringsystem, das substituiert sein kann, bildet
oder R₁ zusammen mit der C-C-Doppelbindung von Formel (IIa) ein 5- bis 8-gliedriges heteroaromatisches Ringsystem, das substituiert sein kann, oder ein 5- bis 8-gliedriges aromatisches Ringsystem, das substituiert sein kann, bildet und
R₂ für H, -CH₃, -CH₂CH₃ oder eine C₃-C₁₀-Alkylgruppe, die linear oder verzweigt sein kann und die teilweise ungesättigt sein kann, steht;
durch eine selektive Reduktion einer Verbindung der Formel (I) 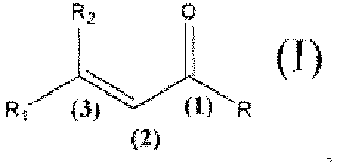 wobei das R, R₁ und R₂ die gleiche Bedeutung wie in der Verbindung der Formel (IIa) definiert haben, **dadurch gekennzeichnet, dass** die selektive Reduktion in Gegenwart mindestens eines Übergangsmetallkatalysators der Formel (III)
[M(L)(X)ₐ(L')_{b}] **(III)**
durchgeführt wird, wobei
M für ein Übergangsmetall steht und
X für ein Anion steht und
L' für einen einzähnigen Liganden steht und
L für einen dreizähnigen Liganden der Formel (IV) 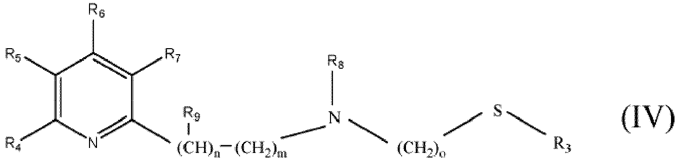 steht, wobei
R₃ für eine lineare C₁-C₄-Alkylgruppe, die substituiert sein kann, eine verzweigte C₃-C₄-Alkylgruppe, die substituiert sein kann, oder eine Phenylgruppe, die substituiert sein kann, steht und
R₄ für H, eine lineare C₁-C₄-Alkylgruppe, die substituiert sein kann, eine verzweigte C₃-C₄-Alkylgruppe, die substituiert sein kann, oder O-C₁-C₂-Alkyl steht und
R₅ für H, eine lineare C₁-C₄-Alkylgruppe, die substituiert sein kann, eine verzweigte C₃-C₄-Alkylgruppe, die substituiert sein kann, oder O-C₁-C₂-Alkyl steht
oder R₄ und R₅ ein C₄-C₈-Ringsystem, das aliphatisch oder aromatisch sein kann, bilden und
R₆ für H, eine lineare C₁-C₄-Alkylgruppe, die substituiert sein kann, eine verzweigte C₃-C₄-Alkylgruppe, die substituiert sein kann, oder O-C₁-C₂-Alkyl steht und
R₇ für H, eine lineare C₁-C₄-Alkylgruppe, die substituiert sein kann, eine verzweigte C₃-C₄-Alkylgruppe, die substituiert sein kann, oder O-C₁-C₂-Alkyl steht und
R₈ für H, eine lineare C₁-C₄-Alkylgruppe, die substituiert sein kann, eine verzweigte C₃-C₄-Alkylgruppe, die substituiert sein kann, steht und
R₉ für -CH₃ oder -CH₂CH₃ steht und
m für 0, 1 oder 2 steht und
n für 0, 1 oder 2 steht,
mit der Maßgabe, dass die Summe von m + n 1 oder 2 beträgt,
o für 2 oder 3 steht,
a für 0, 1, 2 oder 3 steht,
b für 0, 1, 2 oder 3 steht,
mit der Maßgabe, dass die Summe von a + b 2, 3 oder 4 beträgt,
und wobei das Verfahren in Gegenwart mindestens einer Base der Formel (VIII)
M¹(O-C₁-C₅-Alkyl) (VIII)
durchgeführt wird, wobei
M¹ für ein Alkalimetall steht.

2. Verfahren nach Anspruch 1, wobei das Verfahren in Gegenwart mindestens einer Base der Formel (VIII')
M¹ (O-C₃-C₅-Alkyl) (VIII')
durchgeführt wird, wobei
M¹ für Li, Na oder K steht.

3. Verfahren nach Anspruch 1 oder 2, wobei das Verfahren in Gegenwart mindestens einer Base aus der Gruppe bestehend aus KOtBu, NaOtBu und LiOtBu durchgeführt wird.

4. Verfahren nach einem der vorhergehenden Ansprüche, wobei die folgenden Katalysatoren der Formel (III)
[M(L)(X)ₐ(L')_{b}] **(III)**,
wobei
M für ein Übergangsmetall aus der Gruppe bestehend aus Os, Co, Ru und Fe steht und
X für ein Halogen-Anion, ein Carboxylat (wie Acetat oder Benzoat), Borhydrid (wie BH₄⁻), Hydrid, BF₄⁻ oder PF₆⁻ steht und
L' für einen einzähnigen Phosphinliganden steht und
L für einen dreizähnigen Liganden der Formel (IV) 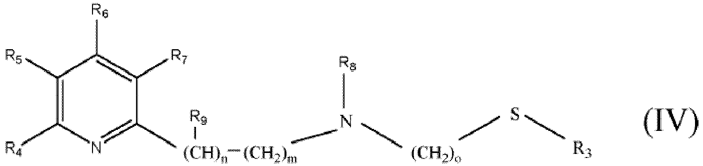 steht, wobei
R₃ für -CH₃ oder -CH₂CH₃ steht und
R₄ für H, -CH₃, -CH₂CH₃, -OCH₃ oder -OCH₂CH₃ steht und
R₅ für H, -CH₃, -CH₂CH₃, -OCH₃ oder -OCH₂CH₃ steht und
R₆ für H, -CH₃, -CH₂CH₃, -OCH₃ oder -OCH₂CH₃ steht und
R₇ für H, -CH₃, -CH₂CH₃, -OCH₃ oder -OCH₂CH₃ steht und
R₈ für H, -CH₃ oder -CH₂CH₃ steht und
R₉ für -CH₃ oder -CH₂CH₃ steht und
oder R₄ und R₅ ein C₄-C₈-Ringsystem, das aliphatisch oder aromatisch sein kann, bilden und
m für 0, 1 oder 2 steht und
n für 0, 1 oder 2 steht,
mit der Maßgabe, dass die Summe von m + n 1 oder 2 beträgt,
o für 2 oder 3 steht,
a für 0, 1, 2 oder 3 steht,
b für 0, 1, 2 oder 3 steht,
mit der Maßgabe, dass die Summe von a + b 2 oder 3 beträgt,
verwendet werden.

5. Verfahren nach einem der vorhergehenden Ansprüche, wobei die folgenden Katalysatoren der Formel (III')
M(L)(X)₂(L') **(III')**,
wobei M für Ru oder Fe steht und
X für Cl⁻ steht und
L' für PPh₃ steht und
L für einen dreizähnigen Liganden aus der Gruppe bestehend aus den Liganden der Formeln (IVa)-(IVl) 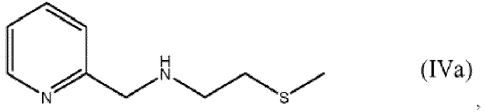 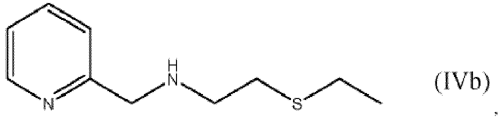 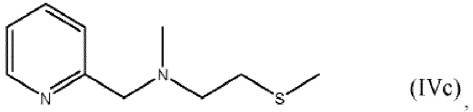 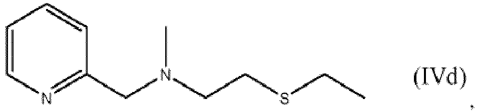 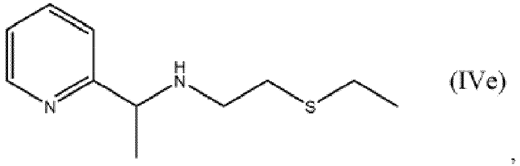 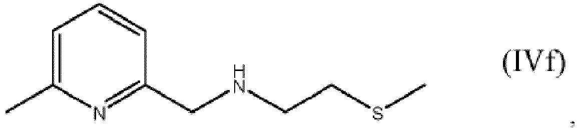 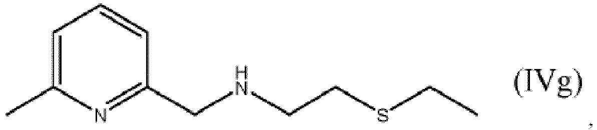 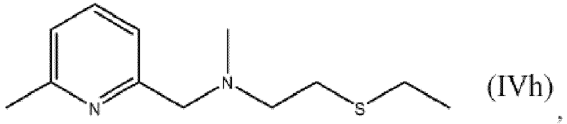 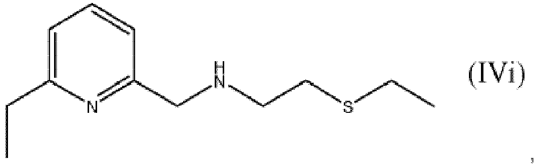 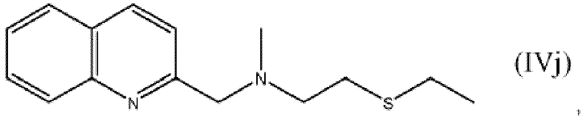 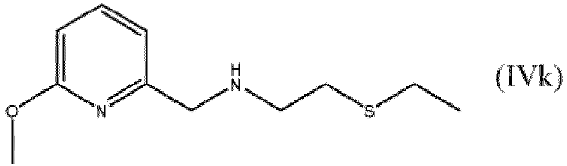 und 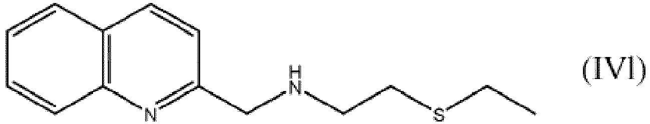 steht,
verwendet werden.

6. Verfahren nach einem der vorhergehenden Ansprüche, wobei der Katalysator der Formel (III) in einer Menge von 0,001-0,5 Mol-% (bezogen auf die Zahl der Mole der Verbindungen der Formel (I)) verwendet wird.

7. Verfahren nach einem der vorhergehenden Ansprüche, wobei es sich bei der Reduktion um eine Transferhydrierung handelt.

8. Verfahren nach einem der Ansprüche 1-6, wobei das Verfahren mit H₂-Gas durchgeführt wird.

9. Verfahren nach Anspruch 8, wobei das Verfahren bei einem Druck von 10 bis 50 bar durchgeführt wird.

10. Verfahren nach einem der vorhergehenden Ansprüche, wobei das Verfahren bei einer Temperatur von 30-150 °C durchgeführt wird.

11. Katalysator der Formel (III)
[M(L)(X)ₐ(L')_{b}] **(III)**,
wobei M für ein Übergangsmetall aus der Gruppe bestehend aus Ru und Fe steht und
X für ein Halogen-Anion (vorzugsweise Cl⁻) steht und
L' für triphenylphosphin steht und
L für einen dreizähnigen Liganden der Formel (IV) 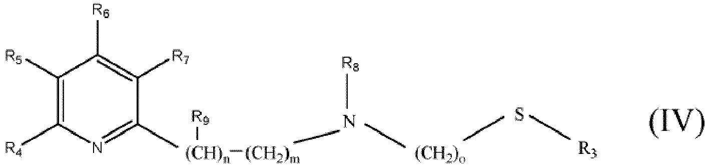 steht, wobei
R₃ für -CH₃ oder -CH₂CH₃ steht und
R₄ für H, -CH₃, -CH₂CH₃, -OCH₃ oder -OCH₂CH₃ steht und
R₅ für H oder -CH₃ steht
oder R₄ und R₅ ein C₄-C₈-Ringsystem, das aliphatisch oder aromatisch sein kann, bilden und
R₆ für H oder -CH₃ steht und
R₇ für H oder -CH₃ steht und
R₈ für H oder -CH₃ steht und
R₉ für -CH₃ steht und
m für 0 oder 1 steht und
n für 0 oder 1 steht,
mit der Maßgabe, dass die Summe von m + n 1 beträgt,
o für 2 steht,
a für 1 oder 2 steht,
b für 1 oder 2 steht,
mit der Maßgabe, dass die Summe von a + b 3 beträgt.

12. Katalysator nach Anspruch 11 mit der Formel (III')
M(L)(X)₂(L') **(III')**,
wobei M für Ru oder Fe steht und
X für Cl⁻ steht und
L' für PPh₃ steht und
L für einen dreizähnigen Liganden aus der Gruppe bestehend aus den Liganden der Formeln (IVa)-(IVl) 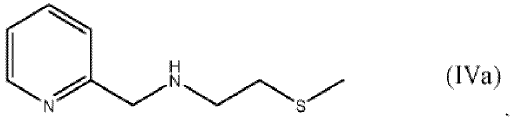 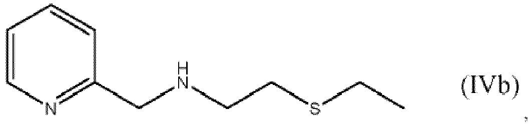 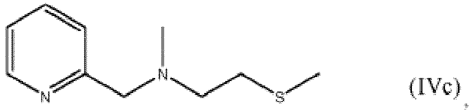 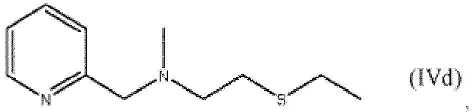 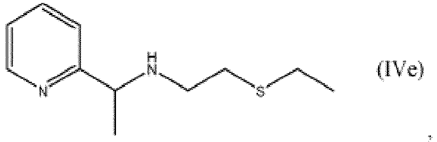 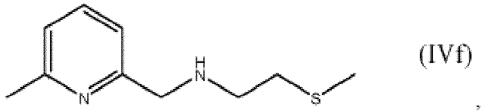 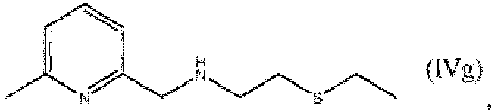 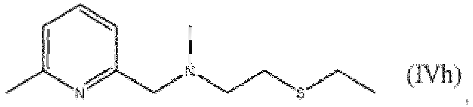 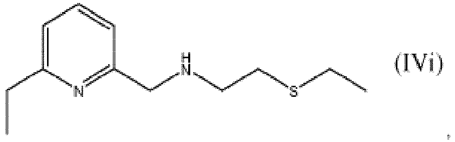 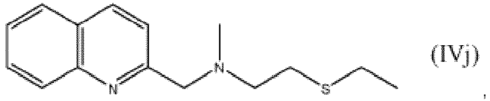 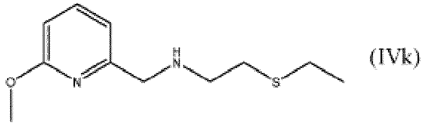 und 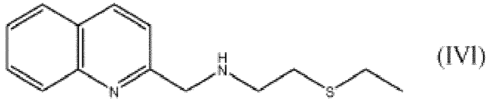 steht.

## Revendications

1. Procédé de production d'un composé de formule (IIa) 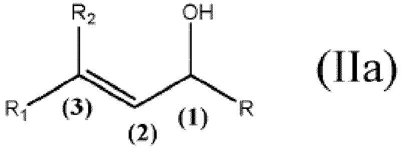
R étant H ou un groupe C₁-₄-alkyle, ou R formant conjointement avec l'atome de carbone (2) un système cyclique aliphatique à 4 à 8 chaînons, qui peut être substitué ; et
R₁ étant H ; un système cyclique aromatique qui est non substitué ou un système cyclique aromatique qui est substitué ; un système cyclique hétéroaromatique qui est non substitué ou un système cyclique hétéroaromatique qui est substitué ; un système cyclique aliphatique qui est non substitué ou un système cyclique aliphatique qui est substitué ; un système cyclique hétéroaliphatique qui est non substitué ou un système cyclique hétéroaliphatique qui est substitué ;-CH₃ ; -CH₂CH₃ ; ou un groupe C₃₋₁₀-alkyle, qui peut être linéaire ou ramifié et qui peut être partiellement insaturé ;
ou R₁ formant conjointement avec la double liaison C-C de la formule (IIa) un système cyclique aliphatique à 5 à 8 chaînons, qui peut être substitué ou un système cyclique aromatique à 5 à 8 chaînons, qui peut être substitué ;
ou R₁ formant conjointement avec la double liaison C-C de la formule (IIa) un système cyclique hétéroaliphatique à 5 à 8 chaînons, qui peut être substitué ou un système cyclique aromatique à 5 à 8 chaînons, qui peut être substitué ;
ou R₁ formant conjointement avec la double liaison C-C de la formule (IIa) un système cyclique aromatique à 5 à 8 chaînons, qui peut être substitué ou un système cyclique aromatique à 5 à 8 chaînons, qui peut être substitué ;
ou R₁ formant conjointement avec la double liaison C-C de la formule (IIa) un système cyclique hétéroaromatique à 5 à 8 chaînons, qui peut être substitué ou un système cyclique aromatique à 5 à 8 chaînons, qui peut être substitué et
R₂ étant H ; -CH₃ ; -CH₂CH₃ ; ou un groupe C₃₋₁₀-alkyle, qui peut être linéaire ou ramifié et qui peut être partiellement insaturé
par une réduction sélective d'un composé de formule (I) 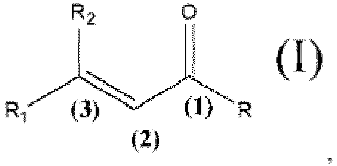 les R, R₁ et R₂ ayant la même signification que définie dans le composé de formule (IIa),
**caractérisé en ce que** la réduction sélective est mise en œuvre en présence d'au moins un catalyseur de métal de transition de formule (III)
[M(L)(X)ₐ(L')_{b}] **(III)**,
M étant un métal de transition et
X étant un anion, et
L' étant un ligand monodentate, et
L étant un ligand tridentate de formule (IV) 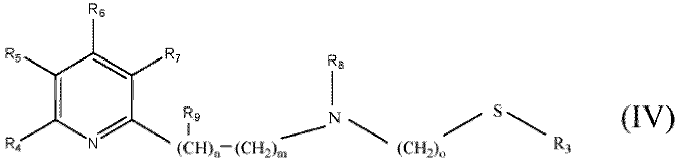
R₃ étant un groupe C₁₋₄-alkyle linéaire, qui peut être substitué ; un groupe C₃₋₄-alkyle ramifié, qui peut être substitué ; ou un groupe phényle, qui peut être substitué, et
R₄ étant H ; un groupe C₁₋₄-alkyle linéaire, qui peut être substitué ; un groupe C₃₋₄-alkyle ramifié, qui peut être substitué ; ou OC₁₋₂-alkyle, et
R₅ étant H ; un groupe C₁₋₄-alkyle linéaire, qui peut être substitué ; un groupe C₃-₄-alkyle ramifié, qui peut être substitué ; ou OC₁₋₂-alkyle,
ou R₄ et R₅ formant un système cyclique en C₄-_{8,} qui peut être aliphatique ou aromatique, et
R₆ étant H ; un groupe C₁₋₄-alkyle linéaire, qui peut être substitué ; un groupe C₃₋₄-alkyle ramifié, qui peut être substitué ; ou OC₁₋₂-alkyle, et
R₇ étant H ; un groupe C₁₋₄-alkyle linéaire, qui peut être substitué ; un groupe C₃₋₄-alkyle ramifié, qui peut être substitué ; ou OC₁₋₂-alkyle, et
R₈ étant H ou un groupe C₁₋₄-alkyle linéaire, qui peut être substitué ; un groupe C₃₋₄-alkyle ramifié, qui peut être substitué, et
R₉ étant -CH₃ ou -CH₂CH₃, et
m étant 0, 1 ou 2, et
n étant 0, 1 ou 2,
étant entendu que la somme de m + n est 1 ou 2,
o étant 2 ou 3,
a étant 0, 1, 2, ou 3,
b étant 0, 1, 2, ou 3,
étant entendu que la somme de a + b est 2, 3 ou 4,
et le procédé étant mis en œuvre en présence d'au moins une base de formule (VIII)
M¹ (OC₁₋₅-alkyle) (VIII)
M¹ étant un métal alcalin.

2. Procédé selon la revendication 1, le procédé étant mis en œuvre en présence d'au moins une base de formule (VIII'),
M¹(OC₃₋₅-alkyle) (VIII')
M¹ étant Li, Na ou K.

3. Procédé selon la revendication 1 ou 2, le procédé étant mis en œuvre en présence d'au moins une base choisie dans le groupe constitué par KOtBu, NaOtBu et LiOtBu.

4. Procédé selon l'une quelconque des revendications précédentes, les catalyseurs suivants de formule (III)
[M(L)(X*)*ₐ(L')_{b}] **(III)**,
M étant un métal de transition choisi dans le groupe constitué par Os, Co, Ru et Fe, et
X étant un anion d'halogène, un carboxylate (tel qu'un acétate ou un benzoate), un borohydrure (tel que BH₄⁻), un hydrure, BF₄⁻, PF₆⁻, et
L' étant un ligand phosphine monodentate, et
L étant un ligand tridentate de formule (IV) 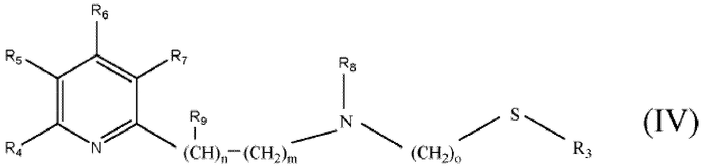
R₃ étant -CH₃ ou -CH₂CH₃, et
R₄ étant H ; -CH₃ ; -CH₂CH₃ ; -OCH₃ ou -OCH₂CH₃, et
R₅ étant H ; -CH₃ ; -CH₂CH₃ ; -OCH₃ ou -OCH₂CH₃, et
R₆ étant H ; -CH₃ ; -CH₂CH₃ ; -OCH₃ ou -OCH₂CH₃, et
R₇ étant H ; -CH₃ ; -CH₂CH₃ ; -OCH₃ ou -OCH₂CH₃, et
R₈ étant H ; -CH₃ ou -CH₂CH₃, et
R₉ étant -CH₃ ou -CH₂CH₃, et
ou R₄, et R₅ formant un système cyclique en C₄-_{8,} qui peut être aliphatique ou aromatique, et
m étant 0, 1 ou 2, et
n étant 0, 1 ou 2,
étant entendu que la somme de m + n est 1 ou 2,
o étant 2 ou 3,
a étant 0, 1, 2, ou 3,
b étant 0, 1, 2, ou 3,
étant entendu que la somme de a + b est 2 ou 3, étant utilisés.

5. Procédé selon l'une quelconque des revendications précédentes, les catalyseurs suivants de formule (III')
M(L)(X)₂(L') **(III')**,
M étant Ru ou Fe, et
X étant Cl⁻, et
L' étant PPh₃, et
L étant un ligand tridentate choisi dans le groupe constitué par les ligands des formules (IVa) à (IVl) 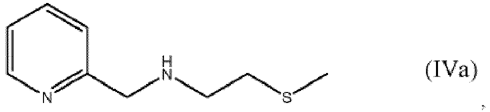 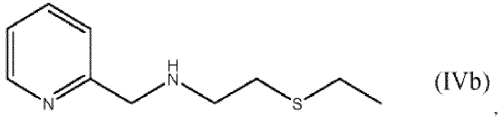 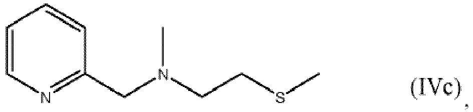 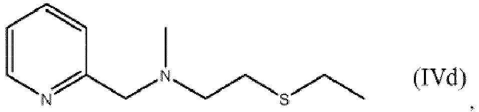 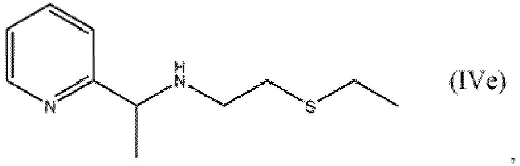 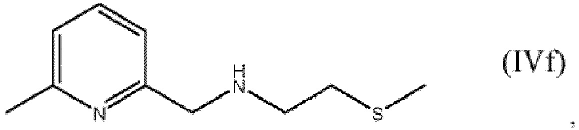 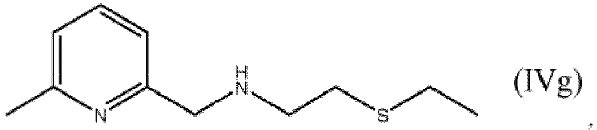 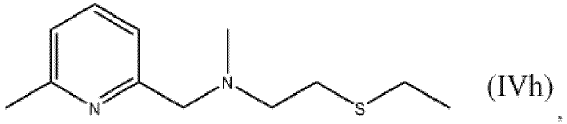 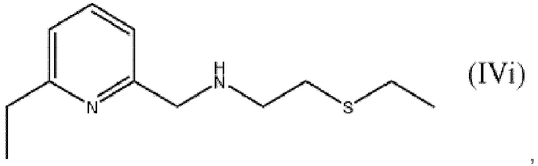 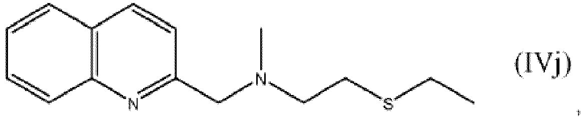 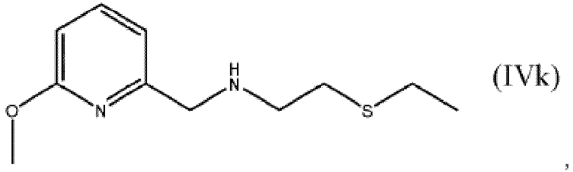 et 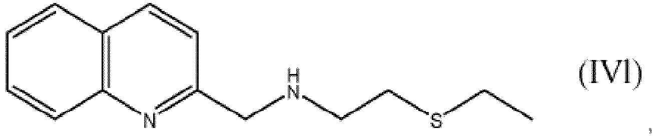 étant utilisés.

6. Procédé selon l'une quelconque des revendications précédentes, le catalyseur de formule (III) étant utilisé en une quantité de 0,001 à 0,5 % en moles (sur la base du nombre de moles des composés de formule (I)).

7. Procédé selon l'une quelconque des revendications précédentes, la réduction étant une hydrogénation par transfert.

8. Procédé selon une quelconque des revendications précédentes 1 à 6, le procédé étant mis en œuvre avec du gaz de H₂.

9. Procédé selon la revendication 8, le procédé étant mis en œuvre à une pression de 10 à 50 bars.

10. Procédé selon l'une quelconque des revendications précédentes, le procédé étant mis en œuvre à une température de 30 à 150 °C.

11. Catalyseur de formule (III)
[M(L)(X)ₐ(L')_{b}] **(III)**,
M étant un métal de transition choisi dans le groupe constitué par Ru et Fe, et
X étant un anion d'halogène (préférablement Cl⁻), et
L' étant triphénylphosphine, et
L étant un ligand tridentate de formule (IV) 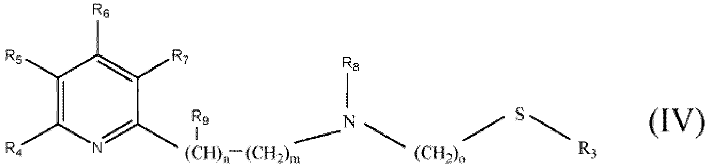
R₃ étant -CH₃ ou -CH₂CH₃, et
R₄ étant H ; -CH₃ ; -CH₂CH₃ ; -OCH₃ ou -OCH₂CH₃, et
R₅ étant H ou -CH₃ ; ou R₄ et R₅ formant un système cyclique en C₄-_{8,} qui peut être aliphatique ou aromatique, et
R₆ étant H ou -CH₃, et
R₇ étant H ou -CH₃, et
R₈ étant H ou -CH₃, et
R₉ étant -CH₃, et
m étant 0 ou 1, et
n étant 0 ou 1,
étant entendu que la somme de m + n est 1,
o étant 2,
a étant 1 ou 2,
b étant 1 ou 2,
étant entendu que la somme de a + b est 3.

12. Catalyseur selon la revendication 11 possédant la formule (III')
M(L)(X)₂(L') **(III')**
M étant Ru ou Fe, et
X étant Cl⁻, et
L' étant PPh₃, et
L étant un ligand tridentate choisi dans le groupe constitué par les ligands des formules (IVa) à (IVl) 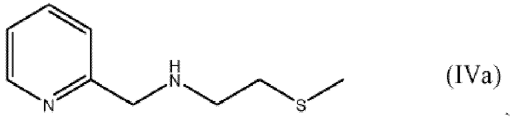 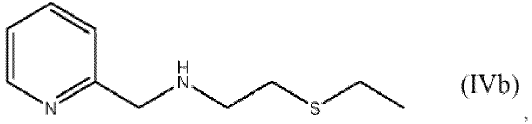 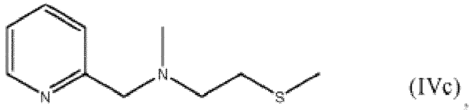 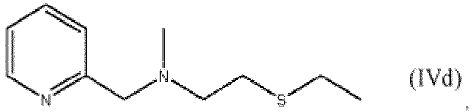 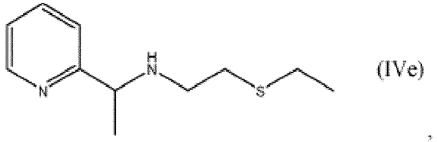 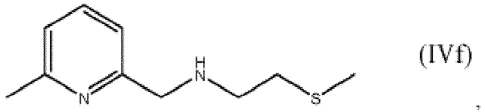 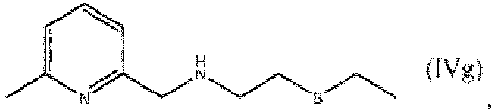 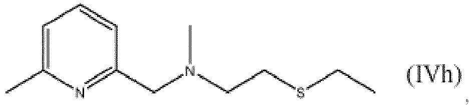 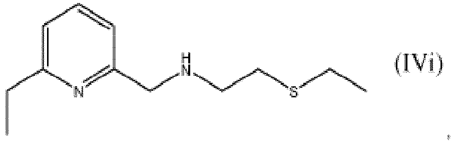 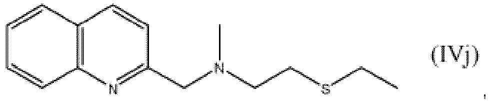 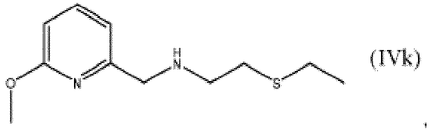 et
